(19) Europäisches Patentamt
European Patent Office
Office européen des brevets

(11) **EP 4 230 143 A1**

(12) **EUROPEAN PATENT APPLICATION**

(43) Date of publication:
**23.08.2023 Bulletin 2023/34**

(21) Application number: **23157493.0**

(22) Date of filing: **20.02.2023**

(51) International Patent Classification (IPC):
**A61B 6/00** *(2006.01)* **G06T 7/11** *(2017.01)*

(52) Cooperative Patent Classification (CPC):
**A61B 6/505; A61B 6/4476; A61B 6/488;**
**G06T 7/11;** A61B 6/0407; A61B 6/547

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **22.02.2022 JP 2022026152**
**20.12.2022 JP 2022203317**

(71) Applicant: **SHIMADZU CORPORATION**
**Kyoto-shi, Kyoto 604-8511 (JP)**

(72) Inventors:
• **OKUMURA, Hiroshi**
**Kyoto, 604-8511 (JP)**
• **YAMAMOTO, Junya**
**Kyoto, 604-8511 (JP)**
• **TAKEDA, Ryo**
**Kyoto, 604-8511 (JP)**

(74) Representative: **Müller-Boré & Partner**
**Patentanwälte PartG mbB**
**Friedenheimer Brücke 21**
**80639 München (DE)**

(54) **X-RAY IMAGING APPARATUS AND IMAGING POSITION CORRECTION METHOD**

(57) An X-ray imaging apparatus (100, 500, 600, 800, 900) includes an X-ray irradiation unit (1, 501), an X-ray detection unit (2), and a correction information acquisition unit (81, 681, 881, 981) configured to identify an outer edge of each of a plurality of predetermined portions of one imaging target of the subject (201) in an X-ray image and acquire position correction information for correcting a relative position of the X-ray irradiation unit with respect to the imaging target of the subject based on a positional relation between the identified outer edges of the plurality of the predetermined portions. The position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged can be captured.

FIG.2

EP 4 230 143 A1

**Description**

**BACKGROUND OF THE INVENTION**

**Field of the Invention**

The present invention relates to an X-ray imaging apparatus and an imaging position correction method.

**Description of the Related Art**

The following description sets forth the inventor's knowledge of related art and problems therein and should not be construed as an admission of knowledge in the prior art.

Conventionally, an X-ray imaging apparatus is known. Such an apparatus is disclosed in, for example, Japanese Unexamined Patent Application Publication No. 2014-117368.

The above-described Japanese Unexamined Patent Application Publication No. 2014-117368 discloses an X-ray imaging apparatus. This X-ray imaging apparatus is provided with an X-ray source for emitting X-rays, an X-ray detector for detecting X-rays, and a display device for displaying a composite moving image acquired by combining an optical moving image and a guiding image. In this X-ray imaging apparatus, an operator instructs the patient to change the imaging position, while viewing the composite moving image displayed on the display on the control room side. Further, the patient changes his/her imaging position while viewing the composite moving image displayed on the display on the imaging room side.

Here, the imaging of an X-ray image in the field of orthopedic surgery is high in difficulty even for an experienced radiological technician. For example, in the case of capturing an X-ray image of a knee joint side face for diagnosing diseases around the knee joint, such as, e.g., disjunctive osteochondral inflammation and osteoarthritis, in order to accurately diagnosing the disease, it is required to capture an X-ray image in which the outer edge of the medial condyle (the mid-line side portion on the femoral knee side) of the femur and the outer edge of the lateral condyle (the opposite side portion opposite t the median line side on the femoral knee side) are overlapped, by adjusting the imaging position.

However, there are differences among individuals in the bone shape and the muscle amount of the foot. For this reason, even for an experienced radiological technician, it is very difficult to perform X-ray imaging such that the outer edge of the medial condyle and the outer edge of the lateral condyle of the femur overlap with each other by adjusting the imaging position from the appearance of the subject. For this reason, in order to capture an X-ray image in which the outer edge of the medial condyle and the outer edge of the lateral condyle of the femur are overlapped, which enables accurate diagnosis, it is necessary to capture an X-ray image and correct the imaging position based on the captured X-ray image.

In order to capture an X-ray image capable of accurately performing diagnosis, it is required to repeatedly perform capturing the X-ray image and correcting the imaging position. Therefore, the exposure dose of the subject and the exposure time increase. Under the circumstances, it is desired to acquire an X-ray image capable of accurately performing diagnosis while suppressing an increase in the exposure dose and the exposure time.

**SUMMARY OF THE INVENTION**

The present invention has been made to solve the above-described problems, and an object of the present invention is to provide an X-ray imaging apparatus and an imaging position correction method capable of obtaining an X-ray image capable of accurately performing diagnosis while suppressing an increase in the exposure dose and the exposure time.

An X-ray imaging apparatus according to a first aspect of the present invention includes:

an X-ray irradiation unit configured to irradiate a subject with X-rays;
an X-ray detection unit configured to detect the X-rays emitted from the X-ray irradiation unit and transmitted through the subject; and
a correction information acquisition unit configured to identify an outer edge of each of a plurality of predetermined portions of one imaging target of the subject in an X-ray image captured based on a direction signal of the X-ray detection unit, and acquire position correction information for correcting a relative position of the X-ray irradiation unit with respect to the imaging target of the subject, based on a positional relation between the identified outer edges of the plurality of the predetermined portions,
wherein the position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged with a predetermined positional relation can be captured.

An imaging position correction method according to a second aspect of the present invention includes:

an irradiation step of irradiating a subject with X-rays from an X-ray irradiation unit;
a detection step of detecting X-rays transmitted through the subject; and
a correction information acquisition step,
wherein the correction information acquisition step identifies an outer edge of each of a plurality of predetermined portions in one imaging target of the subject in an X-ray image captured based on a direction of the X-rays in the detection step, and acquires position correction information for correcting a relative position of the X-ray irradiation unit with respect to the imaging target of the subject, based on a positional relation between identified outer edges of the plurality of predetermined portions, and
wherein the position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged with a predetermined positional relation can be captured.

In the X-ray imaging apparatus according to the first aspect of the present invention and the imaging position correction method according to the second aspect of the present invention, the outer edge of each of the plurality of predetermined portions in one imaging target of the subject in the X-ray image is identified, and the position correction image for correcting the relative position of the X-ray irradiation unit with respect to the imaging target of the subject is acquired, based on the positional relation between the identified outer edges of the plurality of predetermined portions. And the position correction information includes the relative moving direction and the movement amount for correcting the relative position of the X-ray irradiation unit with respect to the imaging target of the subject to the position where an X-ray image in which the positional relation between the outer edges of the plurality of predetermined portions is imaged with a predetermined positional relation can be captured.

With this, the position correction information including the relative moving direction and the movement amount for correcting the relative position of the X-ray irradiation unit with respect to the imaging target of the subject, such as a bone and an artificial joint to a position where the positional relation between the outer edges of the plurality of predetermined portions is imaged with a predetermined positional relation can be captured. Therefore, by notifying the relative moving direction and the movement amount required for the correction by using the acquired position correction information, the user, such as, e.g., a radiological technician, can accurately correct the imaging position based on the notification result. Consequently, it is possible to reduce the number of repetitions of capturing the X-ray image and correcting the imaging position. Accordingly, it is possible to provide an X-ray imaging apparatus and an imaging position correction method capable of acquiring an X-ray image capable of accurately performing diagnosis while suppressing an increase in the exposure dose and the exposure time.

Further, by performing control to change the relative position of the X-ray irradiation unit by using the acquired position correction information, it is possible to accurately correct the relative position of the X-ray irradiation unit with respect to the imaging target of the subject, such as, e.g., a bone and an artificial joint to a position where an X-ray image in which the positional relation between the outer edges of the plurality of predetermined portions is imaged with a predetermined positional relation suitable for diagnosis can be captured. Consequently, it is possible to reduce the number of repetitions of capturing the X-ray image and correcting the imaging position. Accordingly, it is possible to provide an X-ray imaging apparatus and an imaging position correction method capable of acquiring an X-ray image capable of accurately performing diagnosis while suppressing an increase in the exposure dose and the exposure time.

**BRIEF DESCRIPTION OF THE DRAWINGS**

The preferred embodiments of the present invention are shown by way of example, and not limitation, in the accompanying figures.

FIG. 1 is a schematic diagram showing an entire configuration of an X-ray imaging apparatus according to a first embodiment of the present invention.
FIG. 2 is a block diagram showing the configuration of the X-ray imaging apparatus according to the first embodiment of the present invention.
FIG. 3 is a diagram showing one example of a sleeping posture of a subject when imaging a knee joint side.
FIG. 4 shows a bone structure around a right knee joint as viewed from the posterior side.
FIG. 5 shows one example of a main shot image obtained by imaging a knee joint side.
FIG. 6 shows one example of a pre-shot image acquired by imaging a knee joint side.

FIG. 7 is a diagram showing one example of a calculation of a deviation amount using trained models by a processing unit.

FIG. 8 is a graph of a function f(x) at an X-ray tube position x.

FIG. 9 is a diagram showing bones around a knee joint.

FIG. 10 is a diagram showing one example of a display by a display unit of an operation terminal.

FIG. 11 is a first diagram for explaining the correction of the imaging position by the X-ray imaging apparatus according to the first embodiment of the present invention.

FIG. 12 is a second diagram for explaining the correction of the imaging position by the X-ray imaging apparatus according to the first embodiment of the present invention.

FIG. 13 is a diagram showing one example of a method of acquiring position correction information at the time of imaging a humerus.

FIG. 14 is a diagram showing an X-ray image around an elbow joint before and after the position correction.

FIG. 15 shows a processing flow of an imaging position correction in an automatic correction mode of the X-ray imaging apparatus according to the first embodiment.

FIG. 16 is a diagram showing a processing flow of an imaging position correction in a manual correction mode of the X-ray imaging apparatus according to the first embodiment.

FIG. 17 is a diagram for explaining an imaging direction of a scapula.

FIG. 18 is a diagram showing another example of the X-ray imaging apparatus according to the present invention.

FIG. 19 is a diagram showing one example of a display by a display unit of an operation terminal of the X-ray imaging apparatus shown in FIG. 18.

FIG. 20 is a block diagram showing a configuration of an X-ray imaging apparatus according to a second embodiment of the present invention.

FIG. 21 is a diagram for explaining the acquisition of parameter information from a pre-shot image.

FIG. 22 is a diagram showing a processing flow of an imaging position correction method of the X-ray imaging apparatus according to the second embodiment.

FIG. 23 is a block diagram showing the configuration of an X-ray imaging apparatus according to a third embodiment of the present invention.

FIG. 24 is a diagram for explaining the acquisition of parameter information from an appearance image.

FIG. 25 is a block diagram showing the configuration of an X-ray imaging apparatus according to a fourth embodiment of the present invention.

FIG. 26 is a diagram for explaining the acquisition of parameter information from an X-ray image different from a pre-shot image.

## DETAILED DESCRIPTION OF THE PREFERRED EMBODIMENTS

In the following paragraphs, some preferred embodiments of the invention will be described by way of example and not limitation. It should be understood based on this disclosure that various other modifications can be made by those skilled in the art based on these illustrated embodiments.

Hereinafter, some embodiments in which the present invention is embodied will be described with reference to the attached drawings.

### [First Embodiment]

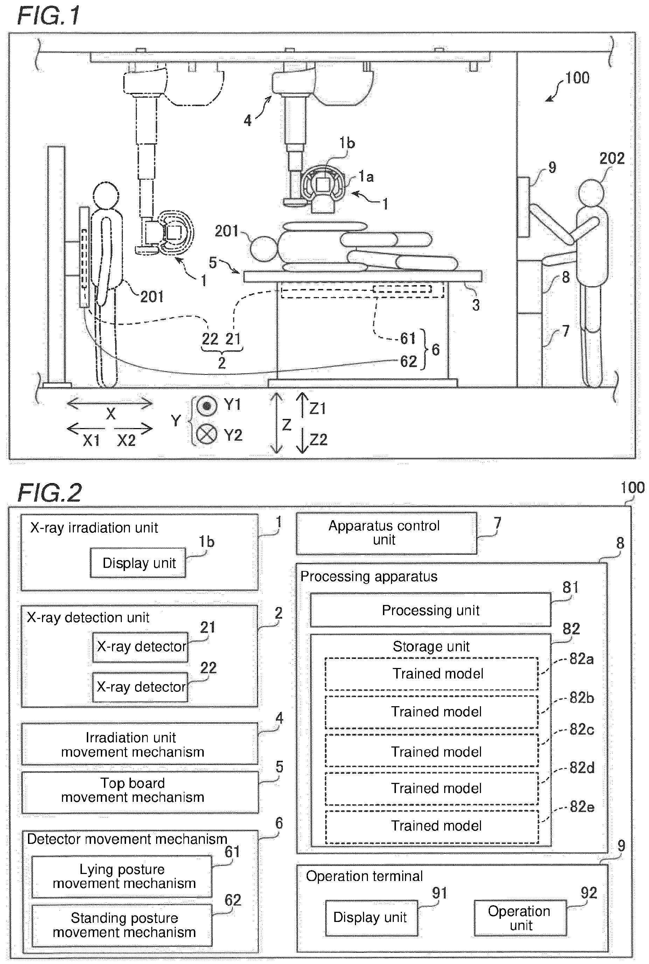

The configuration of the X-ray imaging apparatus 100 according to the first embodiment will be described with reference to FIG. 1 to FIG. 14.

As shown in FIG. 1, the X-ray imaging apparatus 100 is provided with an X-ray irradiation unit 1 and a detection unit 2. Further, the X-ray imaging apparatus 100 is provided with a top board 3 configured to place a subject 201 thereon, an irradiation unit movement mechanism 4, a top board movement mechanism 5, a detector movement mechanism 6, and an apparatus control unit 7. The X-ray imaging apparatus 100 is configured to capture an image by X-rays emitted from the X-ray irradiation unit 1 suspended from a ceiling.

Note that the irradiation unit movement mechanism 4 and the top board movement mechanism 5 are examples of the "movement mechanism" recited in claims. Further, the apparatus control unit 7 is one example of the "movement control unit" recited in claims.

The X-ray irradiation unit 1 is configured to irradiate the subject 201 with X-rays. The X-ray irradiation unit 1 includes an X-ray source (X-ray tube) for irradiating the subject 201 with X-rays, and a collimator for adjusting an irradiation range of X-rays. Further, the X-ray irradiation unit 1 is provided with a gripping portion 1a to be gripped by a user 202 when the user 202 manually moves the X-ray irradiation unit 1. The X-ray irradiation unit 1 is provided with a display unit 1b. The X-ray irradiation unit 1 is configured to be capable of displaying the power assist amount and the imaging conditions by the display unit 1b at the time for the user 202 to move the X-ray irradiation unit 1 by gripping the gripping portion 1a. The display unit 1b is configured to be capable of displaying a position correction information to be described later. The display unit 1b is configured by, for example, a liquid crystal display or an organic EL (electroluminescence) display.

The detection unit 2 is configured to detect X-rays emitted from the X-ray irradiation unit 1 and transmitted through the subject 201. As shown in FIG. 1, the detection unit 2 includes an X-ray detector 21 used to perform imaging in a state in which the subject 201 is laid on the top board 3 (lying posture or lateral posture), and an X-ray detector 22 used to perform imaging in a state in which the subject 201 is in a standing posture (standing position). The X-ray detectors 21 and 22 each are, for example, an FPD (Flat Panel Detector) and detect X-rays transmitted through the subject 201.

The irradiation unit movement mechanism 4 is configured to change the relative position of the X-ray irradiation unit 1 with respect to the subject 201 by moving the X-ray irradiation unit 1. In the X-ray imaging apparatus 100, the X-ray irradiation unit 1 is supported by being suspended from the ceiling by the irradiation unit movement mechanism 4. The X-ray irradiation unit 1 is movably supported in the imaging room by the irradiation unit movement mechanism 4. The irradiation unit movement mechanism 4 is provided with a motor (not shown) and an electromagnetic brake (not shown) corresponding to each of the X-direction, the Y-direction, and the Z-direction. The X-ray irradiation unit 1 is configured to be movable by the irradiation unit movement mechanism 4 in each of the X-direction, the Y-direction, and the Z-direction.

Further, the irradiation unit movement mechanism 4 is provided with an encoder (not shown) used to control the movements of the X-ray irradiation unit 1 corresponding to each of the X-direction, the Y-direction, and the Z-direction. Further, the irradiation unit movement mechanism 4 is provided with a potentiometer (not shown) corresponding to each of the X-direction, the Y-direction, and the Z-direction and is configured to be able to detect the position of the X-ray irradiation unit 1 in each of the X-direction, the Y-direction, and the Z-direction.

The X-ray irradiation unit 1 is configured to be rotatable about the Z-axis. The X-ray irradiation unit 1 is configured to rotate about an axis of each of the axes perpendicular to the Z-axis such as the X-axis and the Y-axis. In the state shown in, e.g., FIG. 1, the X-ray irradiation unit 1 is pivotable about the Y-axis. The X-ray irradiation unit 1 is configured to be able to change the X-ray emission direction and angle by rotating around an axis perpendicular to the Z-axis.

Further, the irradiation unit movement mechanism 4 is provided with a motor (not shown) and an electromagnetic brake (not shown) corresponding to each of the two pivotable axes of the X-ray irradiation unit 1. Further, the irradiation unit movement mechanism 4 is provided with an encoder (not shown) and a potentiometer (not shown), corresponding to each of the two pivotable axes of the X-ray irradiation unit 1. Further, the irradiation unit movement mechanism 4 can detect the absolute position of the X-ray irradiation unit 1 in the vertical direction (Z-direction) by electrically outputting the length of the wire drawn out by moving the X-ray irradiation unit 1.

The top board movement mechanism 5 is configured to change the relative position of the X-ray irradiation unit 1 with respect to the subject 201 by moving the top board 3 to change the position of the subject 201 with respect to the X-ray irradiation unit 1. The top board movement mechanism 5 is provided with a motor (not shown) and an electromagnetic brake (not shown) corresponding to each of the X-direction, the Y-direction, and the Z-direction. The top board 3 is configured to be movable by the top board movement mechanism 5 in each of the X-direction, the Y-direction, and the Z-direction.

The detector movement mechanism 6 includes a lying posture movement mechanism 61 and a standing posture movement mechanism 62. The X-ray detectors 21 and 22 are movably held by the lying posture movement mechanism 61 and the standing posture movement mechanism 62, respectively, in accordance with the imaging site of the subject 201. The lying posture movement mechanism 61 is a device for moving the X-ray detector 21, and is configured to be able to change the position of the X-ray detector 21 with respect to the subject 201. The standing posture movement mechanism 62 is a mechanism for moving the X-ray detector 22 and is configured to change the position of the X-ray detector 22 with respect to the subject 201.

The apparatus control unit 7 is configured to control the entire X-ray imaging apparatus 100. Specifically, the apparatus control unit 7 is configured to perform various controls. The control includes the control of the X-ray irradiation by the X-ray irradiation unit 1, such as, e.g., starting and stopping the X-ray radiation, the control of changing the X-ray irradiation range by the X-ray irradiation unit 1, the control of the detection by the detection unit 2 (X-ray detectors 21 and 22), the control of the movement of the X-ray irradiation unit 1 by the irradiation unit movement mechanism 4, and the control of the movement of the top board 3 by the top board movement mechanism 5. The apparatus control unit 7 is configured to perform the control of the movement of the X-ray detector 21 by the lying posture movement mechanism 61 and the control of the movement of the X-ray detector 22 by the standing posture movement mechanism 62.

The apparatus control unit 7 includes a processor, such as, e.g., a CPU (Central Processing Unit) and an FPGA (Field-Programmable Gate Array). The apparatus control unit 7 is configured to be able to receive detection signals of an encoder and a potentiometer provided to the irradiation unit movement mechanism 4. Further, the apparatus control unit 7 is configured to control the motor and the electromagnetic brake equipped to the irradiation unit movement mechanism 4.

Further, the X-ray imaging apparatus 100 is provided with a processing apparatus 8 including a processing unit 81 and a storage unit 82, as shown in FIG. 2. The processing apparatus 8 is, for example, a PC (Personal Computer) operated by a user 202, such as, e.g., a radiological technician. Further, the processing apparatus 8 is connected to an input device (not shown), such as, e.g., a keyboard and a mouse, and a display device (not shown), such as, e.g., a liquid crystal display and an organic EL display. The processing apparatus 8 is communicatively connected to the apparatus control unit 7. The processing apparatus 8 may be integrally formed with the apparatus control unit 7.

The processing unit 81 is configured to identify an outer edge of each of the plurality of predetermined portions of one bone of the subject 201 in an X-ray image captured based on the detection signal of the detection unit (X-ray detector 21 and X-ray detector 22), and acquire the position correction information for correcting the relative position of the X-ray irradiation unit 1 with respect to the bone of the subject 201, based on the positional relation between the identified outer edges of the specified plurality of predetermined portions. The processing unit 81 includes a CPU, a GPU (Graphics Processing Unit), an ROM (Read Only Memory), and a RAM (Random Access Memory). Note that the processing unit 81 is one example of the "correction information acquisition unit" recited in claims. Further, the bone is one example of the "imaging target" recited in claims.

The position correction information includes the relative moving direction and the movement amount for correcting the relative position of the X-ray irradiation unit 1 with respect to the bone of the subject 201 to a position where an X-ray image in which the positional relation of the outer edges of the plurality of predetermined portions is reflected with a predetermined positional relation can be captured.

The storage unit 82 includes a non-volatile storage medium, such as, e.g., an HDD (Hard Disk Drive) and an SSD (Solid State Drive). The storage unit 82 stores trained models (trained models 82a, 82b, 82c, 82d, and 82e) acquired by machine-training X-ray images of bones as input data.

The trained models 82a, 82b, 82c, 82d, and 82e are, for example, models using, e.g., a U-Net. Note that the machine learning in which an X-ray image is input data may be any one of supervised learning, unsupervised learning, and reinforcement learning. In the first embodiment, the trained model to be stored in the storage unit 82 exists for each of a plurality of predetermined portions of an imaging site. The trained models to be stored in the storage unit 82 have learned a feature point in each of the plurality of predetermined portions of an imaging site. The trained models 82a and 82b are trained models used when imaging the knee joint of the subject 201. The trained models 82c, 82d, and 82e are trained models used when imaging the elbow joint of the subject 201.

Note that the trained models (trained models 82a, 82b, 82c, 82d, and 82e) may be memorized (stored) in a server connected to the X-ray imaging apparatus 100 via a network. The storage unit 82 may store trained models generated for each imaging site.

Further, in the first embodiment, when imaging the knee joint of the subject 201, the processing unit 81 is configured to acquire the outer edges of the plurality of predetermined portions based on the input X-ray image by inputting an X-ray image to the trained models 82a and 82b and calculate the position correction information based on the acquired outer edges of the plurality of predetermined portion. Further, in the first embodiment, when imaging the elbow joint of the subject 201, the processing unit 81 is configured to acquire the outer edges of the plurality of predetermined portions based on the input X-ray image by inputting an X-ray image to the trained models 82c, 82d, and 82e and calculate the position correction information based on the acquired outer edges of the plurality of predetermined portion.

The X-ray imaging apparatus 100 is provided with an operation terminal 9. The operation terminal 9 is a terminal for a user 202 to operate the X-ray imaging apparatus 100. The operation terminal 9 is communicatively connected to the apparatus control unit 7 and the processing apparatus 8. The user 202 can perform the operation for changing the position of the X-ray irradiation unit 1 and for capturing an X-ray image by using the operation terminal 9. The operation terminal 9 is provided with the display unit 91 and the operation unit 92.

The display unit 91 is configured by, for example, a liquid crystal display or an organic EL (electroluminescence) display. Further, the operation unit 92 is a user interface for operating the X-ray imaging apparatus 100. The operation unit 92 includes, for example, a switch, a remote control, or the like. Further, the operation unit 92 may include a touch panel provided to the display unit 91.

The X-ray imaging apparatus 100 is configured to perform the notification of the position correction information acquired by the processing unit 81. In the first embodiment, the X-ray imaging apparatus 100 is configured to perform the notification of the position correction information by displaying on the display unit 91 of the operation terminal 9. Further, in the first embodiment, the X-ray imaging apparatus 100 can perform the notification of the position correction information by displaying on the display unit 1b of the X-ray irradiation unit 1. Note that the detailed description of displaying by the display unit 91 will be described later.

**(Correction at the Time of Imaging Knee Joint)**

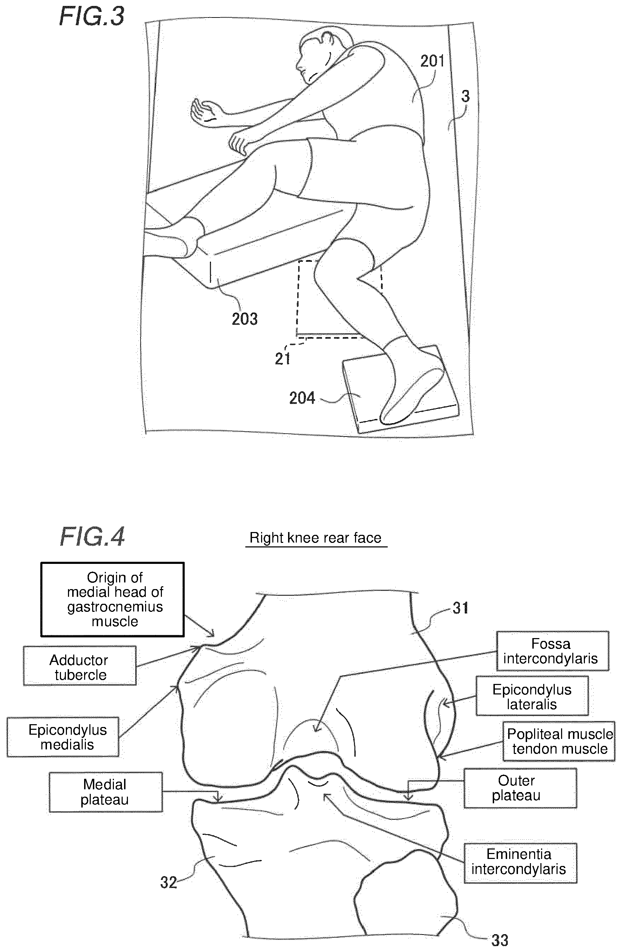

As shown in FIG. 3, when imaging a knee, in a state in which the subject 201 is placed on the top board 3, the sleeping posture of the subject 201 is adjusted so that the position of the knee that is the observation target site, the rotation state of the thigh and the lower leg, the elevation state of the lower leg (bending angle of the knee), and the like become appropriate by using cushions 203 and 204. Then, in the adjusted sleeping posture, an X-ray image of the observation target site of the subject 201 is captured.

The X-ray imaging apparatus 100 is configured to identify (estimate) the plurality of predetermined positions of the femur 31 as shown in FIG. 4 and is configured to acquire the position correction information for correcting the relative position of the X-ray irradiation unit 1 with respect to the bone of the subject 201.

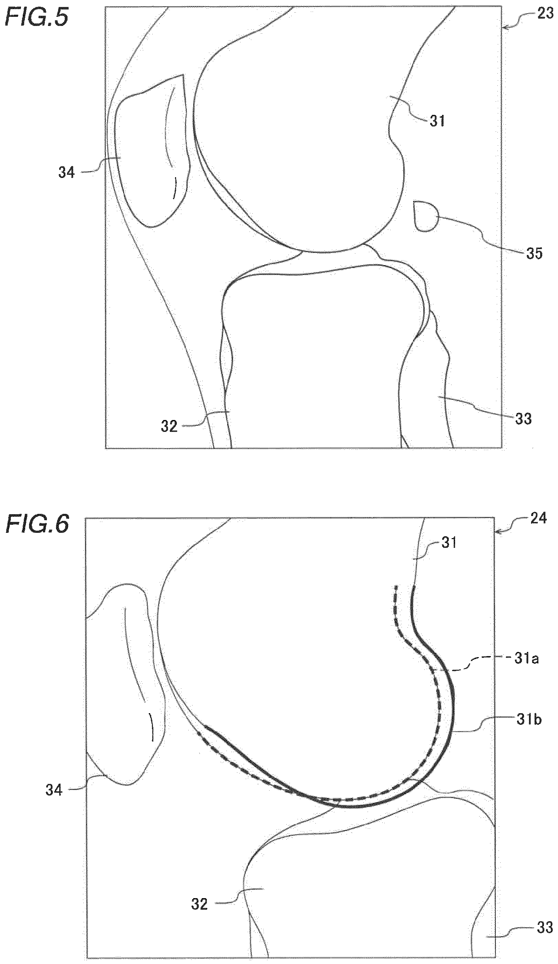

Further, the X-ray imaging apparatus 100 is configured to be able to capture a pre-shot image 24 (see FIG 6) which is an X-ray image generated based on the X-ray radiation with a radiation dose less than that at the time of capturing the main shot image (see FIG. 5) which is an X-ray image captured after correcting the relative position of the X-ray irradiation unit 1 with respect to the bone of the subject 201. For example, the radiation dose at the time of capturing the pre-shot image 24 is about 1/50 to about 1/100 of that at the time of capturing the main shot image 23. In the first embodiment, the main shot image 23 is an image used for diagnosis of diseases.

Note that the main shot image 23 is one example of the "post-position-correction image" recited in claims, and the pre-shot image 24 is one example of the "pre-position-correction image" recited in claims.

Further, in the first embodiment, the processing unit 81 of the X-ray imaging apparatus 100 is configured to acquire the position correction information based on the positional relation of the outer edges of the plurality of predetermined portions of the bone of the subject 201 in the pre-shot image 24.

In the first embodiment, in order to capture the X-ray image in which the outer edge of the medial condyle 31a (see FIG, 6) and the outer edge of the lateral condyle 31b (see FIG. 6) of the femur 31 are overlapped as shown in FIG. 5, the X-ray imaging apparatus 100 is configured to acquire the position correction information by the processing unit 81 and correct the relative position of the X-ray irradiation unit 1 with respect to the bone of the subject 201.

Note that the medial condyle 31a and the lateral condyle 31b are examples of the "plurality of predetermined portions" recited in claims.

The processing unit 81 acquires the relative positional deviation between the outer edges of the plurality of predetermined portions in an X-ray image, based on the positional relation between the outer edges of the plurality of predetermined positions in one bone of the subject 201 in an X-ray image. The processing unit 81 is configured to acquire the position correction information, based on the relative positional deviation between the acquired outer edges of the plurality of predetermined portions. In the first embodiment, at the time of imaging the knee joint side, the processing unit 81 of the X-ray imaging apparatus 100 is configured to acquire the position correction information, based on the positional deviation between the outer edge of the medial condyle 31a (the portion of the the femur 31 on the knee side mid-line side) of the femur 31 and the outer edge of the lateral condyle 31b (the portion of the femur 31 on the opposite side of the the knee side medial line side).

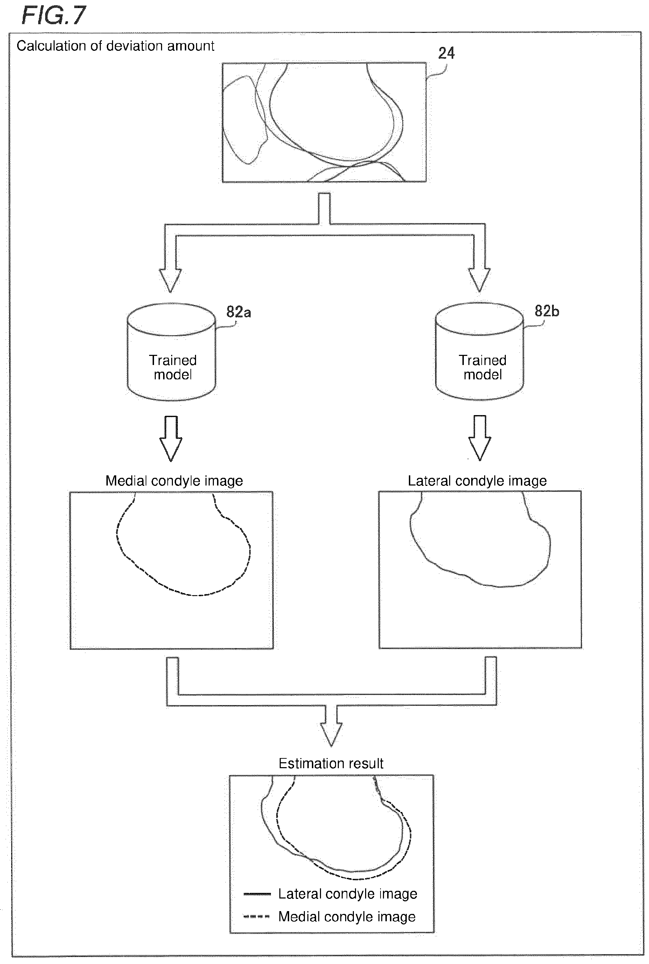

Further, the processing unit 81 is configured to acquire the relative positional deviation between the outer edges of the plurality of predetermined portions, based on the degree of overlap between the outer edges of the plurality of predetermined portions of a bone of the subject 201 in the X-ray image. In the first embodiment, the processing unit 81, as shown in FIG. 7, acquires the relative positional deviation between the outer edges of the plurality of predetermined portions of the knee-side of the femur 31 of the subject 201 by using the trained models 82a and 82b. Specifically, the processing unit 81 identifies (estimates) the outer edge of the medial condyle 31a from the pre-shot image 24 by segmentation processing by the trained model 82a.

Further, the processing unit 81 identifies (estimates) the outer edge of the lateral condyle 3 1b from the pre-shot image 24 by segmentation processing by the trained model 82b. The processing unit 81 is configured to calculate the deviation (the deviation amount and the deviation direction) between the outer edge of the medial condyle 31a and the outer edge of the lateral condyle 31b of the femur 31, based on the outer edge of the medial condyle 31a and the outer edge of the lateral condyle 3 1b identified (estimated) from the pre-shot image 24.

In the analysis of the deviation amount and the deviation direction by the processing unit 81, the deviation amount and deviation direction are determined by virtually moving either the medial condyle 31a or the lateral condyle 31b to search the position at which the area caused by the deviation between the medial condyle 31a and the lateral condyle 31b becomes minimum so that the outer edge of the medial condyle 31a and the rear edge of the outer edge of the lateral condyle 31b coincide. Further, in the analysis of the deviation amount and the deviation by the processing unit 81, the portion where the largest deviation exists may be specified in an image from which the outer edge of each of the medial condyle 31a and the lateral condyle 31b are extracted.

Then, the processing unit 81 is configured to calculate, based on the calculated deviation between the outer edge of the medial condyle 31a and the outer edge of the lateral condyle 31b of the femur 31, the position correction information for correcting the relative position of the X-ray irradiation unit 1 with respect to the bone of the subject 201 to a position where an X-ray image in which the outer edges of the plurality of predetermined portions are overlapped can be captured at the time of imaging the knee-side of the femur 31 of the subject 201.

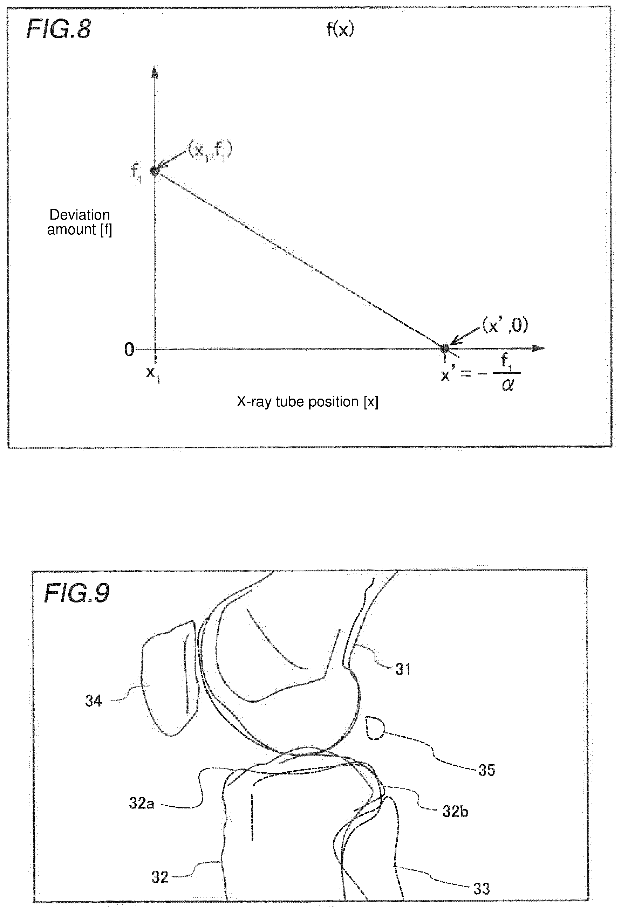

Specifically, the processing unit 81 calculates, as the position correction information, the moving direction and the movement amount (moving distance) of the X-ray irradiation unit 1, based on the calculated deviation (moving vector) between the outer edge of the medial condyle 31a and the outer edge of the lateral condyle 31b of the femur 31. The position correction information is calculated, as shown in FIG. 8, by using the fact that the deviation amount f between the outer edge of the medial condyle 31a of the femur 31 and the outer edge of the lateral condyle 31b of the femur 31 changes linearly with respect to the X-ray tube position x (X-ray irradiation point).

In the first embodiment, the estimated position x' to be estimated as the position (the position at which the deviation amount f becomes 0) at which an X-ray image in which the outer edges of a plurality of predetermined portions (medial condyle 31a and lateral condyle 3 1b) are overlapped can be captured, is calculated based on the deviation amount f 1 between the outer edge of the medial condyle 31a calculated from the pre-shot image 24 captured at the X-ray tube position x1 and the slope $\alpha$ of the function f(x) of the X-ray tube position x. The slope $\alpha$ varies according to various parameters including the parameter of the device, such as, e.g., an SID (Source to Image receptor Distance) and the parameter set corresponding to the subject 201 (patent) at the time of the imaging.

The pre-processing on the pre-shot image 24 may be performed prior to identifying the outer edge of each of the medial condyle 31a and the lateral condyle 31b. For example, processing for removing unwanted signals, such as, e.g., noises, may be performed as pre-processing by a high-pass filter. Further, as the pretreatment, processing of reversing the captured X-ray image of the knee (making the left knee right or making the right knee left) may be performed. In this case, even in a case where only one of the trained models corresponding to one of the right knee and the left knee is stored in the storage unit 82, the processing unit 81 can calculate the deviation amount between the outer edge of the medial condyle 31a and the outer edge of the lateral condyle 31b of both the right knee and the left knee and acquire the position correction information.

Further, the processing unit 81 may perform the identification of the outer edge of each of the medial condyle 31a and the lateral condyle 31b and the calculation of the degree of overlap (positional relation) of the medial condyle 31a and the lateral condyle 31b, based on the bone of the subject 201 other than the femur 31, such as, e.g., a tibia 32, a fibula 33, a patella 34, and a seed bone 35, as shown in FIG. 9, in addition to the features of the medial condyle 31a and the lateral condyle 31b.

For example, in a case where the knee is out-of-crotch (outer crotch), the fibula 33 moves more posteriorly (in a direction away from the patella 34) and the proximal tibiofibular joint becomes clearer. The seed bone 35 is spaced from the condyles (the medial condyle 31a and the lateral condyle 31b) of the femur 31. Further, in the case where the knee is internally turned (inner crotch), the fibula 33 moves further forward (toward the patella 34) to increase the overlap with the tibia 32. The seed bone 35 approaches the condyles (the medial condyle 31a and the lateral condyle 31b) of the femur 31. Therefore, based on the position of the fibula 33 or the position of the seed bone 35, it is possible to determine whether the knee is externally turned (outer crotch) or the knee is internally turned (inner crotch).

Further, in the vertical direction along with the femur 31 extends, in a case where the positional deviation has occurred between the medial condyle 31a and the lateral condyle 31b of the femur 31, a positional deviation is generated between the inner plateau 32a of the tibia 32 and the outer plateau 32b of the tibia 32. Therefore, based on the positional relation between the inner plateau 32a of the tibia 32 and the outer plateau 32b of the tibia 32, it is possible to determine whether there is a positional deviation in the vertical direction between the medial condyle 31a of the femur 31 and the lateral condyle 31b of the femur 31.

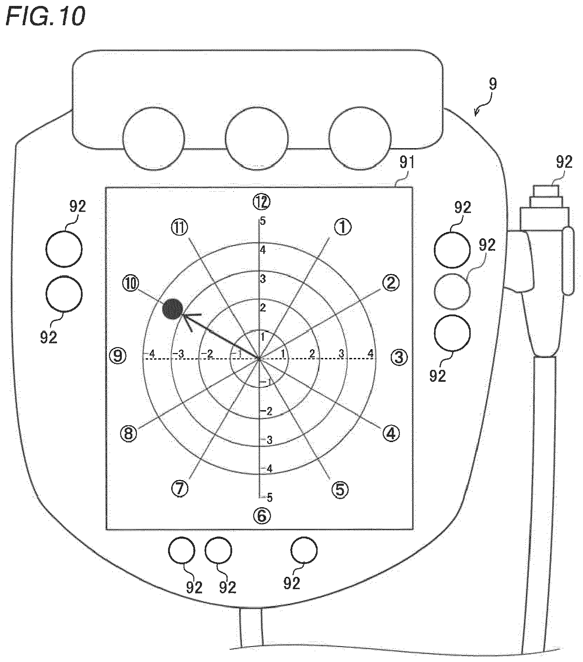

Further, the display unit 91 of the operation terminal 9 displays, as the notification of the position correction information, the relative moving direction and the movement amount of the X-ray irradiation unit 1 with respect to the bone of the subject 201 for correcting the positional relation between the outer edges of the plurality of predetermined portions to a position where an X-ray image in which the positional relation between the outer edges of the plurality of predetermined portions is imaged with a predetermined positional relation can be captured. For example, as shown in FIG. 10, the display unit 91 of the operation terminal 9 displays a chart showing the relative movement amount and movement direction of the X-ray irradiation unit 1 with respect to the bone of the subject 201. As a result, the user 202 can grasp to what extent and in which direction the position of the X-ray irradiation unit 1 with respect to the bone of the subject 201 should be corrected by viewing the display of the display unit 91.

Note that the display by the display unit 91 may be a display using only characters, such as, e.g., "Moving direction: 10 o'clock, Movement amount: 3 cm", or a display combining images, figures, characters, and the like.

The display unit 1b (see FIG. 1) of the X-ray irradiation unit 1 is configured to be displayed in conjunction with the display unit 91. As a result, the user 202 can confirm the relative moving direction and movement amount of the X-ray irradiation unit 1 with respect to the bone of the subject 201 required to correct the imaging position in a state of gripping the gripping portion 1a (see FIG. 1) of the X-ray irradiation unit 1.

**(Control of Movement of X-ray Irradiation Unit)**

The X-ray imaging apparatus 100 is configured to perform control for changing the relative position of the X-ray irradiation unit 1 with respect to the bone of the subject 201 based on the position correction information. Further, in the first embodiment, the X-ray imaging apparatus 100 is provided with an automatic correction mode in which the imaging position is corrected automatically under the control of the apparatus control unit 7 and a manual correction mode in which the imaging position is corrected manually by the user 202. The X-ray imaging apparatus 100 is configured such that the mode for correcting the imaging position can be switched between the automatic correction mode and the manual correction mode, based on the switching operation by the user 202.

In the first embodiment, in the automatic correction mode of the X-ray imaging apparatus 100, the apparatus control unit 7 is configured to perform the automatic correction for automatically changing the relative position of the X-ray irradiation unit 1 with respect to the bone of the subject 201 by moving the position of X-ray irradiation unit 1, based on the position correction information. Further, the X-ray imaging apparatus 100 is configured to restrict the movement of the X-ray irradiation unit 1 at the time of the manual correction (manual correction mode) in which the correction is performed by the movement of the X-ray irradiation unit 1 based on the operation by the user 202.

Specifically, the apparatus control unit 7 performs, in the automatic correction mode, the control of automatically moving the X-ray irradiation unit 1 such that the relative position of the X-ray irradiation unit 1 with respect to the bone of the subject 201 becomes the position capable of capturing the X-ray image in which the positional relation between the outer edges of the plurality of predetermined portions is reflected with a predetermined positional relation, based on the position correction information acquired by the processing unit 81. That is, under the control of the apparatus control unit 7 based on the position correction information, the irradiation unit movement mechanism 4 moves the X-ray irradiation unit 1 to a position where an X-ray image in which the positional relation between the outer edges of the plurality of predetermined portions is imaged with a predetermined positional relation can be captured.

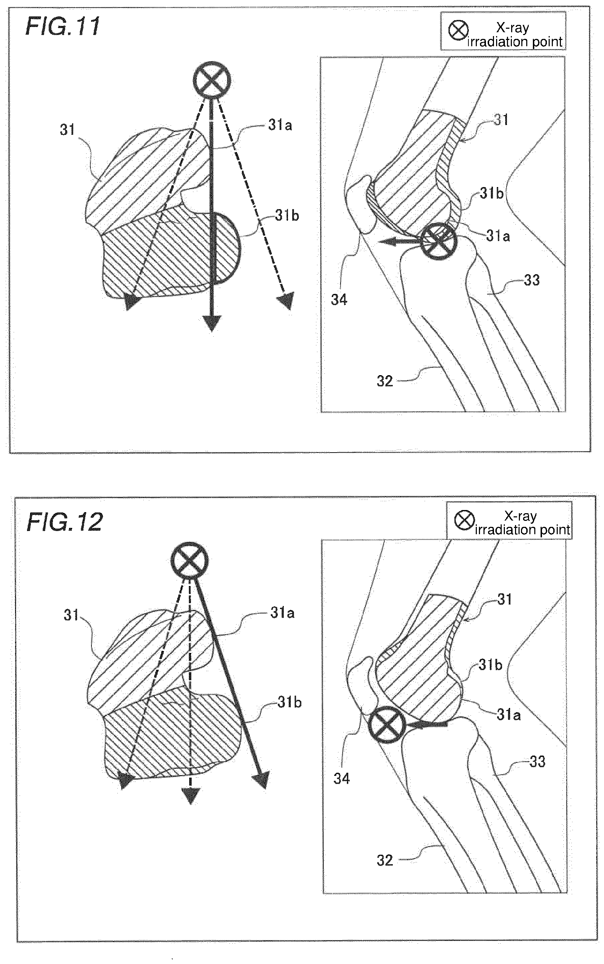

Further, as shown in FIG. 11 and FIG. 12, in the X-ray imaging apparatus 100, by changing the position in the horizontal direction (XY-direction) by fan beam properties of the X-rays emitted from the X-ray irradiation unit 1, it is possible to correct the X-ray irradiation angle of the X-ray irradiation unit 1. For example, by moving the irradiation point (X-ray irradiation unit 1) of X-rays from the position of the left figure of FIG. 11 to the position of the left figure of FIG. 12, X-rays can be emitted to pass the outer edge of the medial condyle 31a of the femur 31 and the outer edge of lateral condyle 31b of the femur 31. Thus, even without changing the X-ray irradiation angle of the X-ray irradiation unit 1, it is possible to capture an X-ray image in a state in which the outer edge of the medial condyle 31a of the femur 31 and the outer edge of the lateral condyle 31b of the femur 31 are overlapped (see the right figure in FIG. 12).

Note that when correcting the imaging position, the X-ray irradiation angle of the X-ray irradiation unit 1 and the position of the X-ray irradiation unit 1 in the vertical direction (Z-direction) may be changed, in addition to the position in the horizontal direction (XY-direction). Further, when correcting the imaging position, the movement of the top board 3 on which the subject 201 is placed may be performed by the top board movement mechanism 5, in addition to the movement of the X-ray irradiation unit 1 by the irradiation unit movement mechanism 4.

Further, the apparatus control unit 7 performs control to restrict the movement of the X-ray irradiation unit 1, based on the position correction information acquired by the processing unit 81 at the time of moving the X-ray irradiation unit 1 based on the operation of the user 202. Specifically, the apparatus control unit 7 is configured to restrict the movement of the X-ray irradiation unit 1 by controlling the locking of the irradiation unit movement mechanism 4 by an electromagnetic brake based on the position correction information in the manual correcting mode.

As described above, in the first embodiment, the apparatus control unit 7 is configured to control the change of the relative position of the X-ray irradiation unit 1 with respect to the bone of the subject 201 by the irradiation unit movement mechanism 4, based on the position correction information acquired by the processing unit 81.

**(Correction At the Time of Imaging Elbow Joint)**

Further, in the first embodiment, the processing unit 81 is configured to acquire the positional relation between the outer edges of the plurality of predetermined portions, based on the positional relation between the outer edges of the plurality of predetermined portions of the bone of the subject 201 in the X-ray image, even at the time of imaging the elbow side (elbow join) of the humerus 36 of the subject 201. In the first embodiment, the processing unit 81 acquires the relative positional deviation between the outer edges of the plurality of predetermined portions on the elbow-side of the humerus 36 of the subject 201 by using the trained models 82c, 82d, and 82e.

Note that when imaging the elbow joint (elbow-side of the humerus 36) of the subject 201, either one of the X-ray detectors 21 and 22 (see FIG. 1) may be used.

In the first embodiment, the processing unit 81 is configured to calculate the position correction information for correcting the relative position of the X-ray irradiation unit 1 with respect to the bone of the subject 201 to the position where an X-ray image in which a plurality of outer edges of a predetermined portion is imaged concentrically can be captured.

Specifically, the processing unit 81 identifies (estimates) each of the portions A, B, and C of the humerus 36 by using the trained models 82c, 82d, and 82e. Then, the processing unit 81 acquires the relative positional deviation between the identified portions A, B, and C of the humerus 36. Further, the processing unit 81 calculates the position correction information for correcting the relative position of the X-ray irradiation unit 1 with respect to the bone of the subject 201 to the position where an X-ray image in which the outer edges of the plurality of predetermined portions (the portions A, B, and C) are imaged concentrically can be captured, based on the acquired relative positional deviation between the portions A, B, and C of the humerus.

In the first embodiment, the processing unit 81 calculates, as the position correction information, the moving direction and the movement amount (moving distance) of the X-ray irradiation unit 11 for correcting the X-ray tube position x (X-ray irradiation point) to the position where an X-ray image in which the outer edges of the portion A and the portion B are overlapped and the outer edges of the plurality of predetermined portions (portions A, B, and C) are reflected concentrically can be captured at the time of imaging the elbow joint.

Note that as the method of calculating the position correction information, the same method as the above-described calculation method at the time of imaging the knee joint is used.

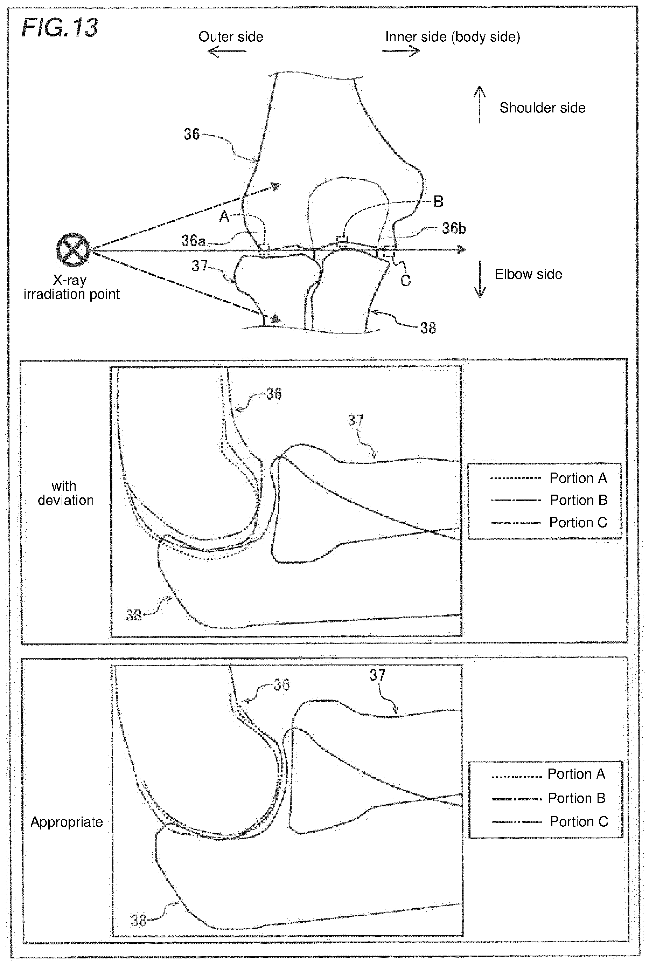

Specifically, as shown in the upper figure of FIG. 13, in the case of capturing an X-ray image by emitting X-rays from the outer side of the humerus 36 toward the humerus capitellum 36a, the position correction information for correcting the relative position of the X-ray irradiation unit 1 with respect to the bone of the subject 201 so that the outer edges of the portion A and the portion C of the humerus 36 on the elbow side are overlapped and the outer edges of the plurality of predetermined portions (the portions A, B, and C) of the humerus 36 on the elbow side are imaged concentrically (see the lower drawing of FIG. 13) is calculated.

The portion A is a convex portion of the humerus capitellum 36a. Further, the portion B is a concave portion of the capitellum 36a of the humerus 36, and the portion C is a convex portion of capitellum 36b of the humerus 36.

Note that the portions A, B, and C are examples of the "plurality of predetermined portions" recited in claims. Note that the calculation of the position correction information at the time of imaging the elbow joint may be performed based on the positional relation between at least one of positional relations of the outer edge of each of the radius 37 and the ulnar 38 and the positional relation between the plurality of predetermined portions (portions A, B, and C) of the humerus 36 on the elbow side.

FIG. 13 shows an example of correcting the position (see the central figure in FIG. 13) where the portion C (the convex portion of the trochlea 6b) of the humerus 36 is imaged with shifted upward to the position to the position (see the below figure in FIG. 13) where the outer edge of each of the portion A (the convex portion of the capitulum of humerus 36a) and the portion C (the convex portion of the trochlea 36b) are overlapped concentrically and the outer edge of the portion B (the concave portion of the trochlea 36b) is imaged concentrically. The relative position of the X-ray irradiation unit 1 with respect to the bone of the subject 201 is corrected from the position in which the portion C (trochlea 36b) of the humerus 36 is imaged with shifted upward (see the middle figure in FIG. 13) by raising the upper arm, raising a table (not shown) on which the upper arm is placed, or changing the position of the X-ray irradiation unit 1, based on the position correction information.

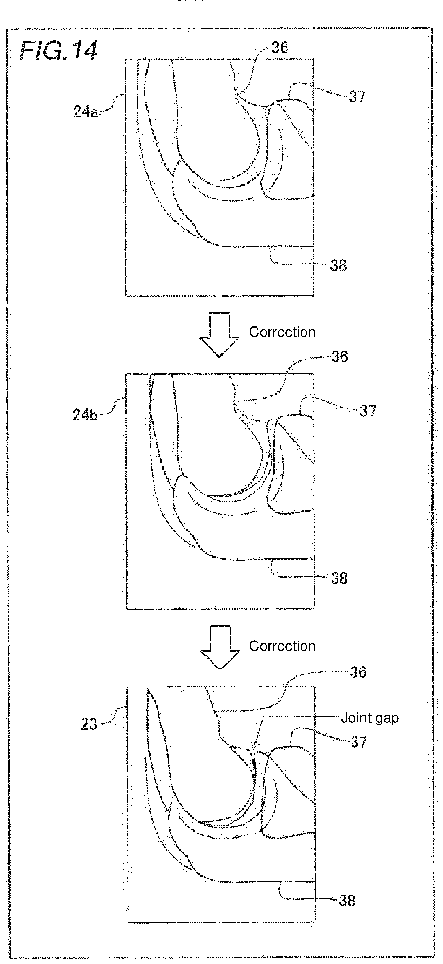

Further, the imaging of the pre-shot image 24 and the correction of the imaging position may be corrected a plurality of times. For example, as shown in FIG. 14, after performing the imaging position based on the pre-shot image 24a of the elbow joint of the subject 201, a pre-shot image 24b may be captured again. Then, the imaging position may be corrected again based on the second pre-shot image 24 (pre-shot image 24b), and the main shot image 23 may be captured. In the example shown in FIG. 14, an X-ray image (main shot image 23) in which the joint gap can be observed between the humerus 36 and the ulnar 38 is captured by capturing the pre-shot images 24 twice and performing the positional correction twice.

**(Position Correction Processing)**

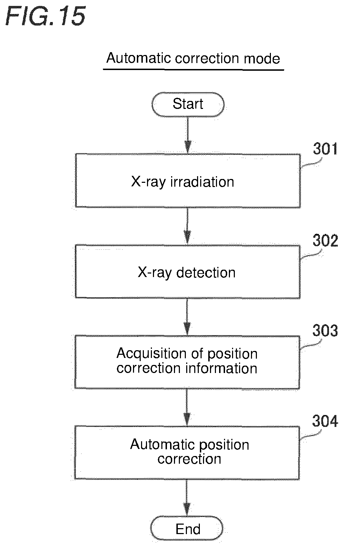

Next, the processing flow of the position correction using an automatic correction mode of the X-ray imaging apparatus 100 according to the first embodiment will be described with reference to FIG. 15.

First, in Step 301, the subject 201 is irradiated with X-rays from the X-ray irradiation unit 1. In Step 301, as described above, X-ray irradiation with less radiation dose than that at the time of capturing the main shot image 23 is performed. Note that Step 301 is one example of the "irradiation step" recited in claims. Then, after completion of Step 301, the processing step proceeds to Step 302.

In Step 302, the X-rays transmitted through the subject 201 are detected. In Step 302, as described above, the X-rays transmitted through the subject 201 are detected by the detection unit 2 (X-ray detectors 21 or 22). As a result, the pre-shot image 24 is captured in Step 302. Note that Step 302 is one example of the "detection step" recited in claims. Then, after completion of Step 302, the processing step proceeds to Step 303.

In Step 303, the position correction information is acquired. In Step 303, as described above, the outer edge of each of the plurality of predetermined portions of the bone of the subject 201 in the X-ray image (pre-shot image 24) to be captured based on the X-ray detection is identified, and the position correction information for correcting the relative position of the X-ray irradiation unit 1 with respect to the bone of the X-ray irradiation unit 1 is acquired based on the positional relation between the specified outer edges of the plurality of predetermined portions.

Note that Step 303 is one example of the "correction information acquisition step" recited in claims. Then, after completion of Step 303, the processing step proceeds to Step 304. The transition to Step 304 may be performed automatically, or may be performed based on the operation by the user 202, such as, e.g., the operation on the operation unit 92 of the operation terminal 9. Also, after completion of Step 303, the position correction information may be displayed on the display unit 91 or the display unit 1b.

Then, in Step 304, the automatic position correction is performed. In Step 304, the control for changing the relative position of the X-ray irradiation unit 1 with respect to the bone of the subject 201 based on the position correction information is performed. Specifically, as described above, the apparatus control unit 7 controls the irradiation unit movement mechanism 4 based on the position correction information to move the X-ray irradiation unit 1. With this, the relative position of the X-ray irradiation unit 1 with respect to the subject 201 (the bone of the subject 201) is corrected. Then, the processing of the position correction using the X-ray imaging apparatus 100 is completed.

Note that Step 304 is one example of the "position correction step" recited in claims.

After completion of Step 304, the user 202 performs the operation for capturing the X-ray image to capture an X-ray image in a state in which the imaging position is corrected. Further, after completion of Step 304, the X-ray image may be automatically captured.

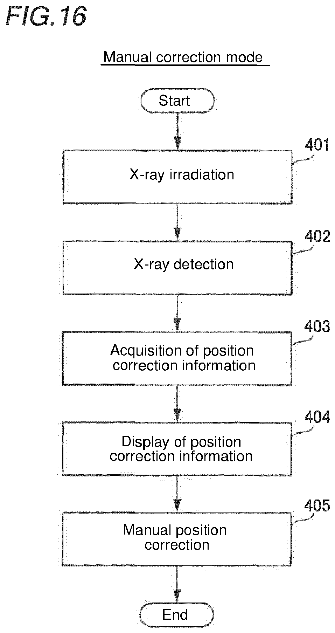

Next, the processing flow of the position correction using the manual correction mode of the X-ray imaging apparatus 100 according to the first embodiment will be described with reference to FIG. 16.

First, in Steps 401, 402, and 403, the same processing as those of Step 301, 302, and 303 in the automatic correction mode is performed. Then, after completion of Step 403, the processing step proceeds to Step 404.

Note that Step 401 is one example of the "irradiation step" recited in claims, and Step 402 is one example of the "detection step" recited in claims. Step 403 is one example of the "correction information acquisition step" recited in claims.

In Step 404, the position correction information is displayed. In Step 404, as described above, the position correction information acquired in Step 403 is displayed by the display unit 91 and the display unit 1b. After displaying the position correction information (after completion of Step 404) by the display unit 91 and the display unit 1b, the processing step proceeds to Step 405. Note that Step 404 is one example of the "position correction step" recited in claims.

In Step 405, a manual position correction is performed. In Step 405, the user 202 corrects the imaging position by moving the X-ray irradiation unit 1 while confirming the display of the display unit 91 or the display unit 1b. In the correction of the imaging position in Step 405, the X-ray irradiation unit 1 may be moved by the hand of the user 202 gripping the gripping portion 1a of the X-ray irradiation unit 1, or may be moved by the irradiation unit movement mechanism 4 based on the operation of the operation terminal 9 on the operation unit 92 by the user 202.

After completion of Step 405, the user 202 performs the operation for capturing an X-ray image to perform the X-ray image in a state in which the imaging position has been corrected.

**(Effects of First Embodiment)**

In the first embodiment, the following effects can be acquired.

In the first embodiment, the position correction information for correcting the relative position of the X-ray irradiation unit 1 with respect to the bone of the subject 201 is acquired based on the positional relation between the outer edges of the plurality of predetermined portions of the bone of the subject 201 in the X-ray image. And the position correction information includes the relative moving direction and the movement amount for correcting the relative position of the X-ray irradiation unit 1 with respect to the bone of the subject 201 to the position where an X-ray image in which the positional relation between the outer edges of the plurality of predetermined positions is imaged with a predetermined relation can be captured.

Accordingly, the position correction information can be acquired. The positional correction information includes the relative moving direction and the movement amount for correcting the relative position of the X-ray irradiation unit 1 with respect to the bone of the subject to the position where an X-ray image in which the positional relation between the outer edges of the plurality of predetermined portions is imaged with a predetermined positional relation can be captured. Therefore, by displaying (notifying) the relative moving direction and the movement amount required for the correction, the user 202, such as, e.g., a radiological technician, can perform the correction of the imaging position with high precision based on the display (notification) result. Consequently, it is possible to reduce the number of repetitions for capturing the X-ray image and correcting the imaging position. As a result, it is possible to provide an X-ray imaging apparatus 100 and an imaging position correction method capable of acquiring an X-ray image capable of accurately performing diagnosis while suppressing the increase in the exposure dose and the exposure time.

Further, by performing the control for changing the relative position of the X-ray irradiation unit 1 by using the acquired position correction information, it is possible to accurately correct the relative position of the X-ray irradiation unit 1 with respect to the bone of the subject 201 to the position where an X-ray image in which the positional relation between the outer edges of the plurality of predetermined portions is imaged with a predetermined positional relation suitable for diagnosis can be captured. Consequently, it is possible to reduce the number of repetitions for capturing of the X-ray image and correcting the imaging position. As a result, it is possible to provide an X-ray imaging apparatus 100 and an imaging position correction method capable of acquiring an X-ray image capable of accurately performing diagnosis while suppressing the increase in the exposure dose and the exposure time.

Further, in the X-ray imaging apparatus 100 according to the first embodiment, the following further advantages can be obtained by configuring as follows.

In the first embodiment, the X-ray imaging apparatus 100 is configured to display (notify) the position correction information acquired by the processing unit 81 (correction information acquisition unit). Accordingly, by the display (notification) of the position correction information acquired by the processing unit 81, the user 202 can grasp the relative moving direction and the movement amount for correcting the relative position of the X-ray irradiation unit 1 with respect to the bone (imaging target) of the subject 201 to the position where an X-ray image in which the positional relation between the outer edges of the plurality of predetermined portions is imaged with a predetermined relation can be captured. Consequently, the user 202 can easily correct the relative position of the X-ray irradiation unit 1 with respect to the bone of the subject 201 to the position where an image in which the positional relation between the outer edges of the plurality of predetermined portions is imaged with a predetermined positional relation can be captured.

Further, the X-ray imaging apparatus 100 is configured to perform control for changing the relative position of the X-ray irradiation unit 1 with respect to the bone of the subject 201 based on the position correction information. With this, based on the position correction information, the control for changing the relative position of the X-ray irradiation unit 1 with respect to the bone of the subject 201 is performed by the X-ray imaging apparatus 100. Therefore, it is possible to easily correct the relative position of the X-ray irradiation unit 1 with respect to the bone of the subject 201 to the position where an X-ray image in which the positional relation between the outer edges of the plurality of predetermined portions is image with a predetermined positional relation can be captured.

Further, in the first embodiment, the apparatus control unit 7 (movement control unit) is configured to control the change of the relative position of the X-ray irradiation unit 1 with respect to the bone (imaging target) of the subject 201 by the irradiation unit movement mechanism 4 (movement mechanism), based on the position correction information acquired by the processing unit 81 (correction information acquisition unit).

With this, the control of changing the relative position of the X-ray irradiation unit 1 with respect to the bone of the subject 201 by the irradiation unit movement mechanism 4 based on the position correction information is performed by the apparatus control unit 7. Therefore, as compared with the case in which the user 202 performs the correction manually based on the position correction information, it is possible to easily perform the correction of the imaging position.

Further, in the first embodiment, the apparatus control unit 7 (movement control unit) performs the control to automatically move the position of the X-ray irradiation unit 1 so that the relative position of the X-ray irradiation unit 1 with respect to the bone (imaging target) of the subject 201 becomes the position where an X-ray image in which the positional relation between the outer edges of the plurality of predetermined portions is imaged with a predetermined positional relation can be captured. With this, the correction of the position of the X-ray irradiation unit 1 is automatically performed, and therefore, the correction of the position of the X-ray irradiation unit 1 can be more easily performed as compared with the case in which the correction of the position of the X-ray irradiation unit 1 is performed by the manual operation of the user 202.

Further, in the first embodiment, the apparatus control unit 7 (movement control unit) performs the control to restrict the movement of the X-ray irradiation unit 1 based on the position correction information acquired by the processing unit 81 (correction information acquisition unit) when the X-ray irradiation unit 1 is moved based on the operation of the user 202. As a result, even in a case where the correction of the imaging position is performed by manually moving the X-irradiation unit 1 by the user 202, the movement of the X-ray irradiation unit 1 is limited by the apparatus control unit 7 based on the position correction information. Consequently, in a case where the imaging position is corrected by manually moving the X-ray irradiation unit 1 by the user 202, it is possible to prevent the movement of the X-ray irradiation unit 1 beyond the movement amount required for the correction and also possible to prevent the movement of the X-ray irradiation unit 1 in a direction other than the moving direction required for the correction.

Further, in the first embodiment, the display unit 91 displays, as a notification of the position correction information acquired by the processing unit 81 (correction information acquisition unit), the relative moving direction and the movement amount of the X-ray irradiation unit 1 with respect to the bone (imaging target) of the subject 201 for correcting the position of the X-ray irradiation unit 1 to the position where an X-ray image in which the positional relation between the outer edges of the plurality of portions is imaged with a predetermined positional relation can be captured. As a result, the user 202 can easily grasp in which direction the correction of the imaging position needs to be corrected by how much by visually recognizing the relative moving direction and the movement amount of the X-ray irradiation unit 1 with respect to the bone of the subject 201 displayed on the display unit 91 to manually correct the imaging position.

Further, in the first embodiment, the processing unit 81 (correction information acquisition unit) is configured to acquire the relative positional deviation between the edges of the plurality of predetermined portions in the X-ray image based on the positional relation between the outer edges of the plurality of predetermined portions in one bone (imaging target) and acquire the position correction information based on the acquired relative positional deviation between the outer edges of the plurality of predetermined portions.

As a result, the relative positional deviation between the outer edges of the plurality of predetermined portions can be acquired from the X-ray image. Accordingly, the positional correction information can be acquired based on the comparison between the relative positional deviation between the outer edges of the plurality of predetermined portions in the X-ray image and the relative positional deviation between the outer edges of the predetermined portions in a state in which the positional relation between the outer edges of the plurality of predetermined portions is in a predetermined positional relation.

Further, in the first embodiment, the processing unit 81 (correction information acquisition unit) acquires the relative positional deviation between the outer edges of the plurality of predetermined portions, based on the degree of overlap between the outer edges of the plurality of predetermined portions of the bone of the subject 201 in the X-ray image. Then, the processing unit 81 calculates the position correction information for correcting the relative position of the X-ray irradiation unit 1 with respect to the subject 201 to the position where an X-ray image in which the the outer edge of the medial condyle 31a and the outer edge of the lateral condyle (outer edges of the plurality of predetermined portions) is imaged in an overlapped manner can be captured.

With this, when imaging the knee side of the femur 31 (around the knee joint) of the femur 31 of the subject 201), the relative position of the X-ray irradiation unit 1 with respect to the bone of the subject 201 can be corrected to the position where an X-ray image in which the outer edge of the medial condyle 31a and the outer edge of the lateral condyle 31b (outer edges of the plurality of predetermined portion) are overlapped can be captured by the position correction information. Consequently, it is possible to capture an X-ray image capable of accurately identifying diseases occurring in the vicinity of the knee joint, such as, e.g., osteochondritis dissecans and knee osteoarthritis.

Further, in the first embodiment, the processing unit 81 (correction information acquisition unit) acquires the relative positional deviation between the outer edges of the plurality of predetermined portions based on the relative positional relation between the outer edges of the plurality of predetermined portions of the bone of the subject 201 in the X-ray image. Then, the processing unit 81 calculates the position correction information for correcting the relative position of the X-ray irradiation unit 1 with respect to the bone of the subject 201 to the position where an X-ray image in which the outer edges of the plurality of redetermined portions (positions A, B, and C) of the outer edge of the humerus 36 are imaged concentrically can be captured at the time of imaging the elbow side of the humerus 36 of the subject 201.

With this, the relative position of the X-ray irradiation unit 1 with respect to the bone of the subject 201 can be corrected to the position where an X-ray image in which the plurality of predetermined portions (portions A, B, and C) of the humerus 36 can be captured by the position correction information when imaging the elbow side (around elbow joint) of the humerus 36 of the subject 201. Consequently, it is possible to captured an X-ray image capable of accurately identifying diseases occurring in the vicinity of the elbow joint, such as, osteochondritis dissecans and knee osteoarthritis.

In the first embodiment, the processing unit 81 (correction information acquisition unit) is configured to acquire the position correction information based on the positional relation between the outer edges of the plurality of predetermined portions of the bone of the subject 201 in the pre-shot image 24 (pre-position-correction image) which is an X-ray image generated based on the X-ray irradiation with less dose than that when capturing the main shot image 23 (post-position-correction image) which is an X-ray image captured after correcting the relative position of the X-ray irradiation unit 1 with respect to the bone (imaging target) of the subject 201. Accordingly, since the X-ray exposure dose of the subject 201 at the time of imaging before the imaging position correction can be reduced, it is possible to further suppress the increase in the exposure dose until an X-ray image capable of performing diagnosis accurately is acquired.

Further, in the first embodiment, the processing unit 81 (correction information acquisition unit) is configured to acquire the outer edges of the plurality of predetermined portions based on the input X-ray image by inputting the X-ray image to the trained models 82a and 82b (trained models 82c, 82d, and 82e) and calculate the position correction information based on the acquired outer edges of the plurality of predetermined portions.

Accordingly, the outer edges of the plurality of predetermined portions are acquired by using the trained models 82a and 82b (trained models 82c, 82d, and 82e), and the position correction information is calculated based on the acquired outer edges of the plurality of predetermined portions. Therefore, by using the trained modules in which feature points of a site of an observation target has been learned, it is possible to acquire the outer edges of the plurality of predetermined portions with high accuracy. Consequently, the position correction information can be calculated with high accuracy.

**[Second Embodiment]**

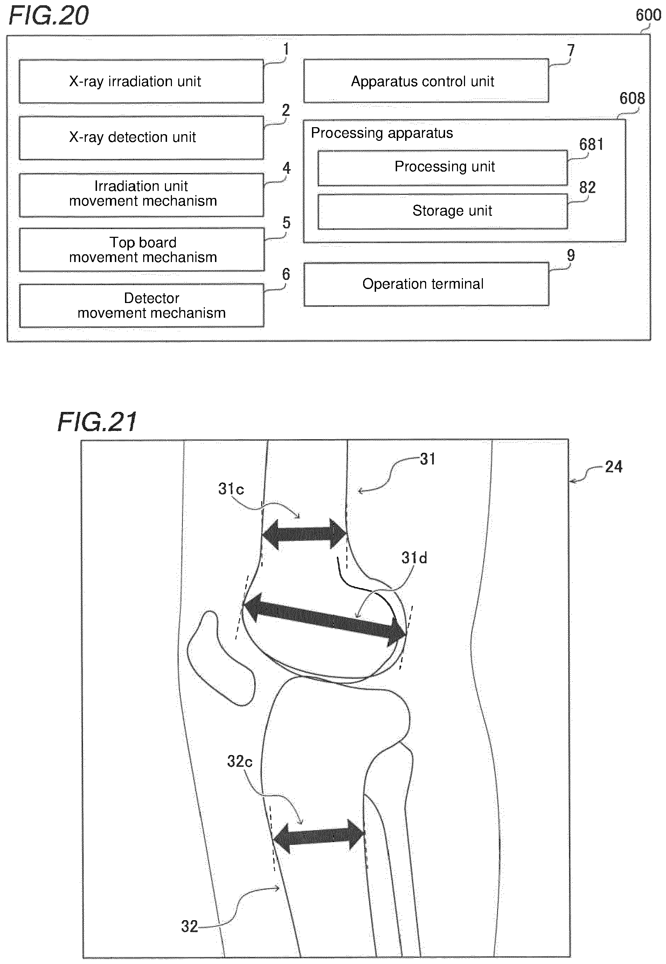

A second embodiment will be described with reference to FIG. 20 and FIG. 21. In this second embodiment, by the processing unit 681, the slope $\alpha$ of the function f(x) at the X-ray tube position x is set and the position correction information is calculated based on the parameter information acquired from the pre-shot image 24. Note that in the figures, the same component as that of the first embodiment will be assigned by the same reference symbol.

**(Configuration X-Ray Imaging Apparatus of Second Embodiment)**

As shown in FIG. 20, the X-ray imaging apparatus 600 is provided with a processing apparatus 608. The processing apparatus 608 includes a processing unit 681. The processing apparatus 608 is, like the processing apparatus 8 of the first embodiment, a PC operated by the user 202, such as, e.g., a radiological technician. The processing unit 681 includes a CPU, a GPU, and a RAM in the same manner as in the processing unit 81 of the first embodiment. Note that the processing unit 681 is one example of the "correction information acquisition unit" recited in claims. In the second embodiment, for example, the X-ray imaging of the subject 201 is performed by the X-ray imaging apparatus 600.

In the same manner as in the processing unit 81 of the first embodiment, the processing unit 681 calculates the deviation (deviation amount and deviation direction) between the outer edge of the medial condyle 31a and the outer edge of the lateral condyle 3 1b of the femur 31 from the pre-shot image 24 when imaging the knee side of the femur 31 of the subject 201. Then, the processing unit 681 calculates, as the position correction information, the moving direction and the movement amount (moving distance) of the X-ray irradiation unit 1 so that imaging can be performed at the imaging position where the outer edges of the medial condyle 31a and the outer edge of the lateral condyle 31b are overlapped, based on the calculated deviation between the outer edge of the medial condyle 31a and the outer edge of the lateral condyle 31b.

At this time, in the same manner as in the first embodiment, the processing unit 681 calculates the estimated position x' at which it is estimated as the position where an X-ray image in which the outer edges of the medial condyle 31a and the lateral condyle 31b are imaged in an overlapped manner can be captured (the position where the deviation amount f becomes 0), based on the deviation amount f 1 between the outer edge of the medial condyle 31a and the outer edge of the lateral condyle 31b calculated from the pre-shot image 24 and the slope $\alpha$ of the function f(x) of the X-ray tube position x, using using the following Formula (1) (see FIG. 8).

$$x' = -\frac{f_1}{\alpha} \quad \cdot \cdot \cdot \quad (1)$$

Here, the slope $\alpha$ varies according to various parameters including the device parameter, such as, e.g., an SID (Source to image receptor distance) and the subject specific parameter set corresponding to the subject 201 (patient) at the time of imaging. Specifically, the slope $\alpha$ is calculated by the following Formula (2).

$$\alpha = -\frac{b + D \cdot \cos\theta}{SID - (b + D \cdot \cos\theta)} + \frac{b}{SID - b} \quad \cdot \cdot \cdot \quad (2)$$

Where the SID is the vertical axis position of the X-ray tube as a device parameter and is a distance from the detection surface of the detection unit 2 (X-ray detector 21 and X-ray detector 22) to the X-ray irradiation unit 1. The SID may be automatically set as a recommended value according to the site or the like of the object to be imaged, or may be acquired from the actual value from the apparatus control unit 7.

b" indicates the height from the detection surface to the lateral condyle 31b. "D" indicates the actual distance between the medial condyle 31a and the lateral condyle 31b in the subject 201. "$\theta$" indicates the slope of the straight line connecting the medial condyle 31a and the lateral condyle 31b with respect to the perpendicular line from the detection surface. The heights b, the distance D, and the angle $\theta$ are subject specific parameters. The height b and the distance D are parameters derived from the physique that varies depending on the physique (bone size) of the subject 201. The angle $\theta$ is a parameter derived from the posture that varies with the posture of the subject 201.

The processing unit 681 according to the second embodiment automatically calculates the parameters derived from the physique from the pre-shot image 24. That is, the slope $\alpha$ is automatically calculated based on the pre-shot image 24.

In the second embodiment, the processing unit 681 calculates the position correction information, based on the parameter information corresponding to the actual distance between the medial condyle 31a and the lateral condyle 31b (a plurality of predetermined portions) of the bone (imaging target) of the subject 201. The processing unit 681 detects, as the parameter information, the size of the detection target portion of the subject 201 in the X-ray image of the subject 201. Specifically, the processing unit 681 detects, as the parameter information, the size of the detection target portion of the subject 201 from the pre-shot image 24, which is an X-ray image taken to acquire the position correction information. Then, the processing unit 681 calculates the position correction information by calculating the height b and the distance D based on the detected parameter information.

As shown in FIG. 21, the detection target portion of the subject 201 is, for example, the portion 31c and the portion 31d of the femur 31, and the portion 32c of the tibia 32 of the subject 201. In the second embodiment, the detection target portion (portion 31c, portion 31d, and portion 32c) of the subject 201 whose size is detected as parameter information is a portion that differs from the plurality of predetermined portions (medial condyle 31a and lateral condyle 31b) in the imaging target of the subject 201, which is an object to be imaged with a predetermined positional relation. The detection target portion is a portion that correlates to the size or the shape of the predetermined portion.

The processing unit 681 performs segmentation processing by algorithm, such as, e.g., trained models stored in advance, to detect, as the parameter information, the size (width) of each of the portion 31c of the femur 31 and the portion 32c of the tibia 32 of the subject 201, from the pre-shot image 24. For example, the processing unit 681 uses trained models to detect the contour line of each of the femur 31 and the tibia 32 in pre-shot image 24.

The processing unit 681 detects the size (number of pixels) of the width of the portion 31c, the portion 31d, and the portion 32c from the detected contour line to detect the size of the target portion of the subject 201. The position of the portion 31c, the portion 31d, and the portion 32c in the pre-shot image 24 may be extracted from the shape of the contour line, the position coordinate, or the like, or may be acquired based on the input operation by the user 202.

The processing unit 681 calculates the height b and the distance D for calculating the position correction information from the detected size of the portion 31c, the portion 31d, and the portion 32c detected as parameter information by referring to, for example, a preset data table. In the preset data table, each of the size of the portion 31c and the portion 31d of the femur 31 of the subject 201 and the portion 32c of the shinbone, the height b from the detection surface to the lateral condyle 3 1b, and the actual distance D between the medial condyle 31a and the lateral condyle 31b are stored in association with each other.

That is, the processing unit 681 calculates the height b and the distance D, which are parameter information derived from the physique unique to the subject 201, by acquiring the size of the portion 31c and the portion 31d of the femur 31, and the portion 32c of the tibia 32 of the subject 201, which are detection target portions detected as parameter information.

By using the height b and the distance D acquired as described above, the processing unit 681 calculates the slope α using the above-described Formula (2). Note that the angle θ which is a posture-derived parameter may be automatically set to the value recommended in accordance with the site of the object to be imaged, or may be detected by detecting the posture of the subject 201 using an optical camera or the like. Then, the processing unit 681 calculates the position correction information corresponding to the size and the shape of the bone that differs for each subject 201, by calculating the estimated position x' by the above-described Formula (1) using the slope α reflecting the parameter derived from the physique.

The rest of the configuration of X-ray imaging apparatus 600 according to the second embodiment is the same as that of the first embodiment. That is, the acquisition of the pre-shot image 24, the acquisition of the deviation amount f1 from the pre-shot image 24, and the processing of the position correction based on the calculated position correction information are the same as those of the first embodiment.

**(Imaging Position Correction Method According to Second Embodiment)**

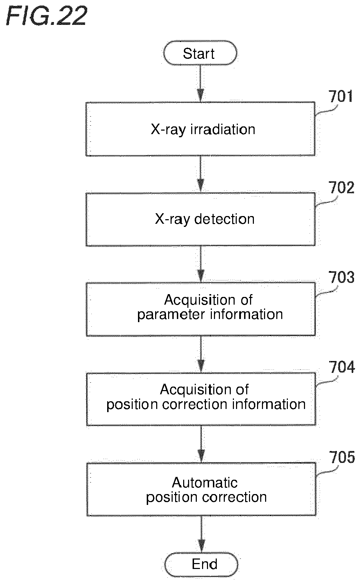

Next, with reference to FIG. 22, the processing flow of the imaging position correction method of the X-ray imaging apparatus 600 according to the second embodiment will be described. In FIG. 22, the processing flow of the position correction using the automatic correction mode will be described.

First, in Step 701 and Step 702, processing similar to those of Step 301 and 302 in the first embodiment are performed, respectively. Then, after completion of Step 702, the processing step proceeds to Step 703. Note that Step 701 is one example of the "irradiation step" recited in claims. Step 702 is one example of the "detection step" recited in claims.

In Step 703, parameter information corresponding to the actual distance between the medial condyle 31a and the lateral condyle 31b (the plurality of predetermined portions) of the bone (imaging target) of the subject 201 is acquired. Specifically, as the parameter information, the size of each (the detection target portion) of the portion 31c of the femur 31 and the portion 31d, and the portion 32c of the tibia 32 is detected from the pre-shot image 24 captured in Step 702.

Then, in Step 704, the position correction information is calculated based on the parameter information. Specifically, the height b and the distance D are acquired based on the sizes of the portion 31c and the portion 32c of the femur 31 and the portion 32c of the tibia 32 acquired as parameter information. Then, the slope α is calculated based on the acquired heights b and distance D, and the position correction information is acquired based on the acquired deviation (the positional relation between the outer edges of the plurality of predetermined portions) acquired from the pre-shot image 24.

Note that Step 704 is one example of the "correction information acquisition step" recited in claims.

Then, in Step 705, the automatic position correction is performed in the same manner as in Step 304 of the first embodiment. Note that Step 704 is one example of the "position correction step" recited in claims.

Note that in the case of executing the processing of the position correction using the manual correction mode, in the same manner as in the first embodiment, after the position correction information is acquired by performing the same processing as in Steps 701, 702, 703, and 704 in the automatic correction mode, the display of the position correction information is performed in the same manner as in Step 404 of the first embodiment, and the manual position correction is performed in the same manner as in Step 405.

**(Effects of Second Embodiment)**

In the second embodiment, the following effects can be obtained.

The processing unit 681 (correction information acquisition unit) is configured to calculate the position correction information, based on the parameter information corresponding to the actual distance between the plurality of predetermined portions (the medial condyle 31a and the lateral condyle 31b) of the bone (imaging target) of the subject 201.

With this configuration, even in a case where the actual distance between the predetermined portions of the bone of the subject 201 differs due to the fact that the size and the shape of the bone differ for the subject 201, it is possible to more accurately calculate the position calculation information by calculating the position correction information based on the parameter information corresponding to the actual distance between the predetermined portions. Therefore, the imaging position can be corrected with higher accuracy by using the acquired position correction information. Therefore, it is possible to further reduce the number of repetitions for capturing the X-ray image and correcting the imaging position. Consequently, the increase in the exposure dose and the exposure time in the X-ray imaging can be further suppressed.

The processing unit 681 (correction information acquisition unit) detects, as parameter information, the size of the detection target portion (the portion 31c and the portion 31d of the femur 31, the portion 32c of the tibia 32) of the subject 201 in the X-ray image of the subject 201.

With this configuration, by detecting, as the parameter information, the size of the detection target portion of the subject 201 from the X-ray image, it is possible to acquire the parameter information so that the difference of the size and the shape of the femur 31 and the tibia 32, which are structures in the body of the subject 201, is more accurately reflected. Therefore, by acquiring the position correction information using the parameter information detected from the X-ray image of the subject 201, the relative moving direction and the movement amount required for correcting the imaging position can be acquired more accurately. Consequently, the correction of the imaging position can be performed more accurately.

The processing unit 681 (correction information acquisition unit) detects, as parameter information, the size of the detection target portion (the portion 31c and the portion 31d of the femur 31 and the portion 32c of the tibia 32) of the subject 201, from the pre-shot image 24 which is an X-ray image captured to acquire the position correction information.

With this configuration, the parameter information can be acquired by using the pre-shot image 24 captured to acquire the position correction information. Therefore, unlike the case where an X-ray imaging is performed separately from the capturing of the pre-shot image 24 in order to acquire the parameter information, it is possible to suppress an increase in the exposure dose of the X-rays emitted to the subject 201. Therefore, by acquiring the parameter information from the pre-shot image 24, it is possible to perform the correction of the imaging position more accurately and further suppress the increase in the exposure dose and the exposure time in the X-ray imaging.

Other effects of the second embodiment are the same as those of the first embodiment.

**[Third Embodiment]**

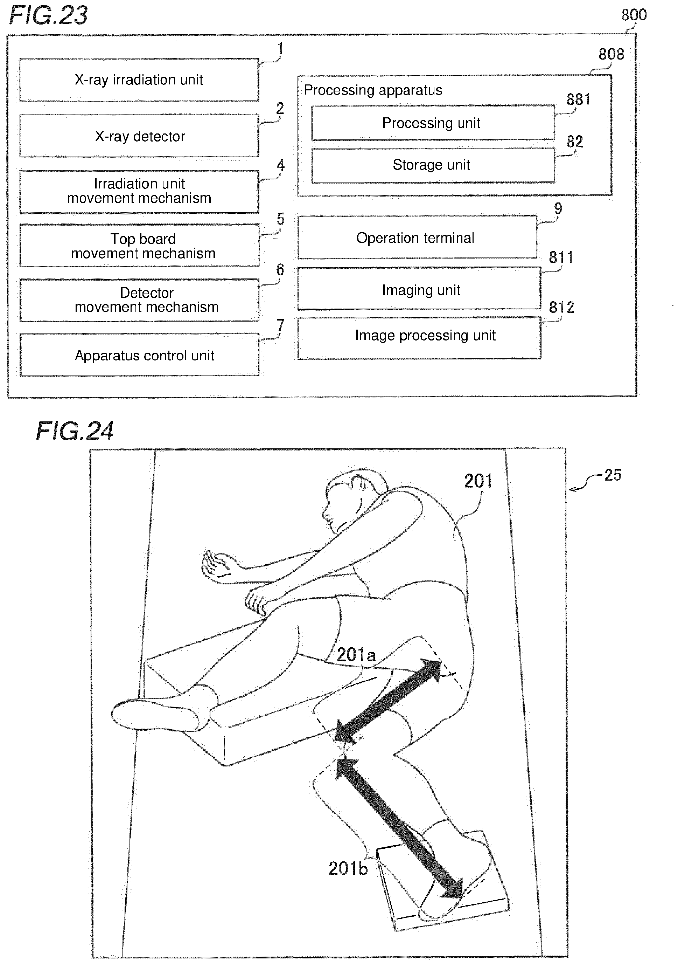

With reference to FIGS. 23 and 24, a third embodiment will be described. In this third embodiment, unlike the second embodiment configured to detect, as the parameter information for setting the slope $\alpha$, the size of the detection target (the portion 31c and the portion 31d of the femur 31, the portion 32c of the tibia 32) of the subject 201 form the pre-shot image 24, the parameter information for setting the slope $\alpha$ is acquired based on the appearance image 25.

In the figures, the same components as those of the first and second embodiment are assigned by the same reference symbols.

**(Configuration X-Ray Imaging Apparatus of Third Embodiment)**

As shown in FIG. 23, the X-ray imaging apparatus 800 is provided with a processing apparatus 808. The processing apparatus 808 includes a processing unit 881. The processing apparatus 808, like the processing apparatus 608 of the second embodiment, is, for example, a PC operated by the user 202, such as, e.g., a radiological technician. The processing unit 881, like the processing unit 681 of the second embodiment, includes a CPU, a GPU, and a RAM. The processing unit 881 is one example of the "correction information acquisition unit" recited in claims. In the same manner as in the processing unit 681 of the second embodiment, the processing unit 881 automatically calculates the slope $\alpha$ by the above-described Formula (2). In the third embodiment, for example, X-ray imaging of the knee of the subject 201 is performed by the X-ray imaging apparatus 800.

In the third embodiment, the X-ray imaging apparatus 800 is provided with an imaging unit 811 and an image processing unit 812. The imaging unit 811 images the appearance of the subject 201. The imaging unit 811 is arranged, for example, in the X-ray irradiation unit 1.

The imaging unit 811 images the subject 201 lying on the top board 3, along the illumination direction of the X-rays emitted from the X-ray irradiation unit 1. Further, the imaging unit 811 includes an image sensor (imaging element), such as, e.g., a CCD (Charge Coupled Device) and a CMOS (Complementary Metal Oxide Semiconductor).

The image processing unit 812 includes, for example, a processor, such as, e.g., a CPU and an FPGA. The image processing unit 812 includes a nonvolatile storage medium, such as, e.g., an HDD or an SSD for storing various parameters and programs. The image processing unit 812 acquires the appearance image 25 (see FIG. 24) acquired by imaging the appearance of the subject 201 by acquiring the signal from the imaging unit 811. The image processing unit 812 is a module provided separately from the processing unit 881. The image processing unit 812 is arranged in the X-ray irradiation unit 1 together with, for example, the imaging unit 811. The image processing unit 812 is configured to be communicable with the processing unit 881.

Then, as shown in FIG. 24, in the third embodiment, the image processing unit 812 calculates the physique information indicating the size of the physique of the subject 201 from the appearance image 25 of the subject 201 acquired by imaging with the imaging unit 811, as the parameter information for calculating the slope $\alpha$ in the above-described Formula (2). Specifically, the image processing unit 812 calculates the length of the leg of the subject 201 as the physique information.

The image processing unit 812 detects the lengths of the portion 201a and the portion 201b of the leg of the subject 201 from the appearance image 25 acquired based on the signal from the imaging unit 811. The portion 201a is the part of the subject 201 from the base of the leg to the knee. The portion 201b is the part from the knee to the toe of the leg of the subject 201. The image processing unit 812 detects the lengths of the portion 201a and the portion 201b by extracting the portion 201a and the portion 201b of the leg of the subject 201 from the appearance image 25, by using, for example, trained models previously stored in the storage medium. With this, the image processing unit 812 detects, as the parameter information, the physique information indicating the length of the leg of the subject 201 from the appearance image 25.

Then, in the same manner as in the processing unit 681 of the second embodiment, the image processing unit 812 refers to, for example, a data table set in advance in the storage medium, and calculates the height b and the distance D for calculating the position correction information from the physique information indicating the length of the leg of the subject 201 detected as the parameter information. In the preset data table, the physique information, the height b from the detection surface to the lateral condyle 3 1b, and the actual distance D between the medial condyle 31a and the lateral condyle 31b are stored in association with each other.

That is, in the third embodiment, the image processing unit 812 calculates the height b and the distance D, which are parameters derived from the physique inherent to the subject 201, by acquiring the physique information as the parameter information. In the second embodiment, the height b and the distance D calculated by the image processing unit 812 are output to the processing unit 881 of the processing apparatus 808.

By using the height b and the distance D acquired as described above, the processing unit 881 calculates the the value of the slope $\alpha$ by the above-described Formula (2) in the same manner as in the processing unit 681 of the first embodiment, and calculates the estimated position x' by the above-described Formula (1), thereby calculating the position correction information corresponding to the size and the shape of bone that differ for each subject 201. That is, in the third embodiment, the processing unit 881 is configured to calculate the position correction information, based on the parameter information, which is the physique information calculated from the appearance image 25 by the image processing unit 812.

Note that the rest of the configuration of the X-ray imaging apparatus 800 according to the third embodiment is the same as that of the first and second embodiments described above.

**(Effects of Third Embodiment)**

In the third embodiment, the following effects can be acquired.

In the third embodiment is provided with an imaging unit 811 for imaging the exterior of the subject 201. The processing unit 881 (correction information acquisition unit) calculates the position correction information, based on the parameter information, which is the physique information indicating the size of physique of the subject 201 calculated from the appearance image 25 of the subject 201 acquired by the imaging by the imaging unit 811.

With this configuration, the parameter information which is the physique information indicating the size of the physique of the subject 201 can be acquired based on the appearance image 25, and therefore, it is possible to acquire the position correction information corresponding to the physique size of the subject 201. Therefore, it is possible to more accurately acquire the relative moving direction and the movement amount required for correcting the imaging position so as to correspond to the physique size of the subject 201, and therefore, the correction of the imaging position can be performed more accurately.

The other effects of the third embodiment are the same as those of the first and second embodiments.

**[Fourth Embodiment]**

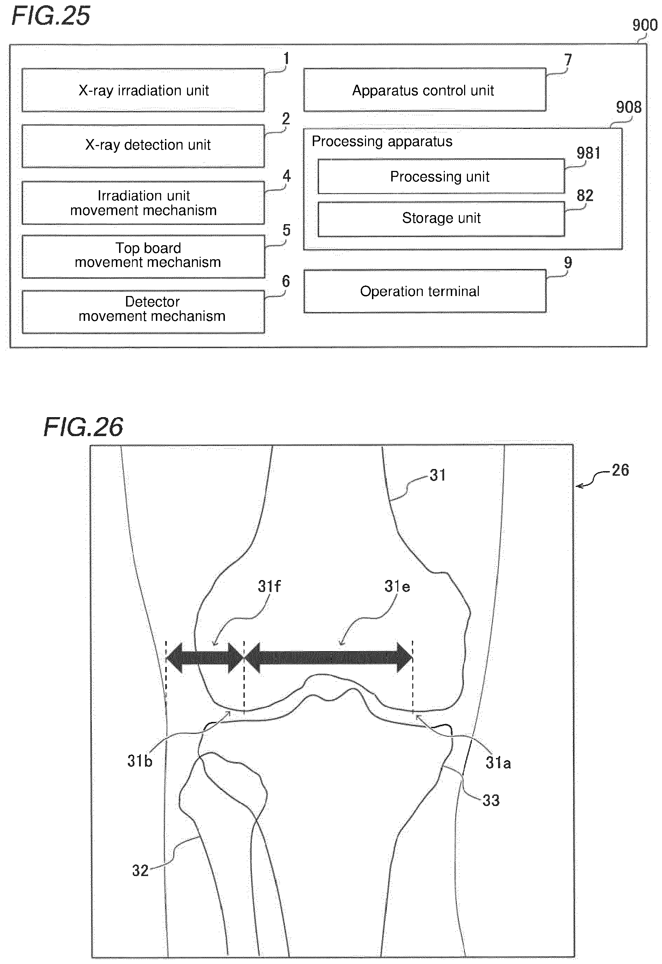

With reference to FIGS. 25 and 26, a fourth embodiment will be described. In this fourth embodiment, unlike the second embodiment configured to acquire the parameter information from the pre-shot image 24 by the processing unit 681, the parameter information for setting the slope $\alpha$ is be acquired by the processing unit 981, based on the front image 26, which is an X-ray image previously acquired separately from the pre-shot image 24. Note that in the figures, the same component as that of the first to third embodiments are assigned by the same reference numeral.

**(Configuration X-Ray Imaging Apparatus of Fourth Embodiment)**

As shown in FIG. 25, the X-ray imaging apparatus 900 is provided with the processing apparatus 908. The processing apparatus 908 includes a processing unit 981. The processing apparatus 908 is, like the processing apparatus 608 of the second embodiment, for example, a PC operated by the user 202, such as, e.g., a radiological technician. The processing unit 981 includes, like the processing unit 681 of the second embodiment, a CPU, a GPU, and a RAM. Similarly to the processing unit 681 of the second embodiment, the processing unit 981 automatically calculates the slope $\alpha$ by the above-described Formula (2).

Note that the processing unit 981 is one example of the "correction information acquisition unit" recited in claims. In the fourth embodiment, for example, the X-ray imaging of the knee of the subject 201 is performed by the X-ray imaging apparatus 900.

As shown in FIG. 26, in the fourth embodiment, the processing unit 981 acquires a front image 26 that is an X-ray image captured in advance at an imaging angle that differs from the pre-shot image 24, which is an X-ray image for acquiring the position correction information.

Then, the processing unit 981 detects, as the parameter information for calculating the slope $\alpha$, the size of the detection target portion of the subject 201 from the front image 26. Specifically, the front image 26 is an X-ray image in which the knee of the subject 201 is captured in advance from the front side and separately from the pre-shot image 24 in which the knee of the subject 201 is imaged from the lateral side of the subject 201. The front image 26 is captured at the timing prior to the pre-shot image 24 and stored in the storage unit 82 in advance. The detection target portions of the subject 201 to be detected from the front image 26 are the portion 31e from the medial condyle 31a to the lateral condyle 31b of the femur 31 of the subject 201 and the portion 31f from the lateral condyle 31b to the skin of the subject 201 opposite to the median line. Therefore, in the fourth embodiment, the detection target portion of the subject 201 whose size is detected as the parameter information is a plurality of predetermined portions of the imaging target of the subject 201, which is an object to be imaged with a predetermined positional relation.

The processing unit 981 detects the positions of the medial condyle 31a and the lateral condyle 3 1b from the front image 26 captured in advance prior to capturing the pre-shot image 24.

For example, the processing unit 981 extracts the contour of the femur 31 by the segmentation processing using the trained models stored in advance and detects, as the position of the medial condyle 31a and the lateral condyle 31b, the position closest to the tibia 32 in the medial condyle 31a and the portion closest to the tibia 32 in the lateral condyle 31b, among the extracted contour line of the femur 31. The processing unit 981 detects the size (distance) from the position of the medial condyle 31a to the position of the lateral condyle 31b, as the size of the portion 31e which is the detection target portion of the subject 201.

Similarly, by detecting the position of the skin of the subject 201 in the front image 26, the processing unit 981 detects the size (distance) from the position of the lateral condyle 31b to the position of the skin, as the size of the portion 3 1f which is the detection target portion of the subject 201.

Here, the size of the portion 31e corresponds to the actual distance D between the medial condyle 31a and the lateral condyle 31b. The size of the portion 31f corresponds to the height b from the detection surface to the lateral condyle 31b. That is, in the fourth embodiment, the processing unit 981 directly detects the values of the height b and the distance D as the parameter information from the front image 26 separately captured at the imaging angle different from the pre-shot image 24.

In the same manner as in the processing unit 681 of the second embodiment, the processing unit 981 calculates the slope α by using the above-described Formula (2) by using the height b (the size of the portion 31f) from the detection surface detected as the parameter information from the lateral condyle 31b and the actual distance D (the size of the portion 31e) between the medial condyle 31a and the lateral condyle 31b, and calculates the position correction information corresponding to the size and the shape of the bone that differ for each subject 201.

Note that the rest of the configuration of the X-ray imaging apparatus 900 according to the fourth embodiment is the same as that of the first to third embodiments described above.

**(Effects of Fourth Embodiment)**

In this fourth embodiment, the following effects can be obtained.

The processing unit 981 (correction information acquisition unit) detects, as the parameter information, the size of the detection target portion (the portion 31e and the portion 31f) of the subject 201 from the front image which is an X-ray image previously captured at an imaging angle different from that of the pre-shot image 24 which is an X-ray image for acquiring the position correction information.

With this configuration, by detecting the size of the detection target portion from the X-ray image (front image 26) captured at the imaging angle different from the X-ray image (pre-shot image 24) for correcting the position, it is possible to detect the positional relation between the plurality of predetermined portions (the medial condyle 31a and the lateral condyle 3 1b) from the imaging angle different from the pre-shot image 24. Therefore, it is possible to acquire more accurate position correction information based on the positional relation between the plurality of predetermined portions from the imaging angle different from the pre-shot image 24.

Note that the other effects of the fourth embodiment are the same as those of the first to third embodiments.

**[Modified Embodiment]**

It should be understood that the embodiments disclosed here are examples in all respects and are not restrictive. The scope of the present invention is shown by the claims rather than the descriptions of the embodiments described above, and includes all changes (modifications) within the meaning equivalent to claims.

For example, in the first to fourth embodiments described above, an example is shown in which the processing unit 81, 681, 881, and 981 (correction information acquisition unit) is configured to acquire the position correction information for correcting the relative position of the X-ray irradiation unit 1 with respect to the bone of the subject 201 based on the positional relation between the outer edges of the plurality of predetermined portions of the bone of the subject 201 in the X-ray image, but the present invention is not limited thereto.

In the present invention, it may be configured such that the correction information acquisition unit acquires the position correction information for correcting the relative position of the X-ray irradiation unit with respect to the artificial joint of the subject based on the positional relation between the outer edges of the plurality of predetermined portions of one artificial joint of the subject in the X-ray image. Further, it may be configured such that the correction information acquisition unit of the X-ray imaging apparatus acquires the position correction information both at the time of imaging the bone and at the time of imaging the artificial joint.

Note that the artificial joint is one example of the "imaging target" recited in claims.

Further, in the first to fourth embodiments, an example is shown in which the display of the position correction information acquired by the processing unit 81, 681, 881, and 981 (correction information acquisition unit) and the control for changing the relative position of the X-ray irradiation unit 1 with respect to the bone of the subject 201 based on the position correction information are performed, but the present invention is not limited thereto.

In the present invention, the X-ray imaging apparatus may only perform the notification of the position correction information acquired by the correction information acquisition unit. In this case, the user, such as, e.g., a radiological technician, manually corrects the imaging position based on the notified position correction information. Further, the correction of the relative position of the X-ray irradiation unit with respect to the bone or the artificial joint (imaging target) of the subject may be performed by moving the subject by the user or by the movement of the subject. Further, it may be configured such that the X-ray imaging apparatus is configured to automatically correct the imaging position without notifying the position correction information acquired by the correction information acquisition unit.

Further, in the above-described first to fourth embodiments, an example is shown in which the apparatus control unit 7 (movement control unit) automatically moves the position of the X-ray irradiation unit 1 so that the relative position of the X-ray irradiation unit 1 with respect to the bone of the subject 201 becomes the position where an X-ray image in which the positional relation between the outer edges of the plurality of predetermined portions is imaged with a predetermined positional relation can be captured, based on the position correction information acquired by the processing unit 81, 681, 881, and 981, but the present invention is not limited thereto.

Further, in the present invention, when correcting the relative position of the X-ray irradiation unit with respect to the bone or the artificial joint (imaging target) of the subject, the correction of the imaging position may be performed by automatically moving both the X-ray irradiation unit and the top board on which the subject is to be placed. Further, when correcting the relative position of the X-ray irradiation unit for the bone or the artificial joint (imaging target) of the subject, only the top board on which the subject is to be placed may be automatically moved to the correct imaging position.

Further, in the above-described first to fourth embodiments, an example is shown in which the apparatus control unit 7 (movement control unit) performs the control for limiting the movement of the X-ray irradiation unit 1, based on the position correction information acquired by the processing unit 81, 681, 881, and 981 (correction information acquisition unit), but the present invention is not limited thereto.

In the present invention, the X-ray imaging apparatus may be configured not to restrict the movement of the X-ray irradiation unit while the user is operating. Further, the X-ray imaging apparatus may be configured to perform the restriction of the movement and the release of the restriction of the movement of the X-ray irradiation unit based on the the user's operation. Further, in the present invention, the movement control unit may perform the control to restrict the movement of the top board on which a subject is to be placed, based on the position correction information acquired by the correction information acquisition unit when moving the top board on which a subject is to be placed, based on the user's operation. For example, the movement control unit may restrict the movement of the top board by the movement mechanism by performing the control to lock the movement of the top board by a brake.

With this, the movement of the top board on which a subject is to be placed is restricted by the movement control unit based on the position correction information even in a case where the correction of the imaging position is performed by manually operating the movement of the top board on which a subject is to be placed by the user. Consequently, in the case of correcting the imaging position by manually operating the movement of the top board on which a subject is to be placed by the user, it is possible to prevent the top board on which a subject is to be placed from being moved exceeding the movement amount required for the correction and the top board on which a subject is to be placed from being moved in a direction other than the moving direction required for the correction.

In the above-described first to fourth embodiments, an example is shown in which the display unit 91 shows the relative moving direction and the movement amount of the X-ray irradiation unit 1 with respect to the bone of the subject 201, based on the relative positional deviation between the outer edges of the plurality of predetermined portions acquired by the processing unit 81 (681, 881, 981), as a notification of the position correction information acquired by the processing unit 81, 681, 881, 981 (moving direction), but the present invention is not limited thereto.

In the present invention, the display unit may display only the relative moving direction of the X-ray irradiation unit among the relative moving direction and the movement amount of the X-ray irradiation unit with respect to the bone of the subject. The X-ray imaging apparatus completes the correction of the imaging position by limiting (locking) the movement of the X-ray irradiation unit when the X-ray irradiation unit moved by the user's manual operation has reached the appropriate imaging position acquired based on the position correction information.

Further, in the above-described first to fourth embodiments, an example is shown in which when imaging the knee joint (femur 31), the relative position of the X-ray irradiation unit 1 with respect to the bone or the artificial joint (imaging target) of the subject 201 is corrected to the position where an X-ray image in which the outer edges of the plurality of predetermined portions are imaged in an overlapped manner can be captured, by the position correction information, but the present invention is not limited thereto.

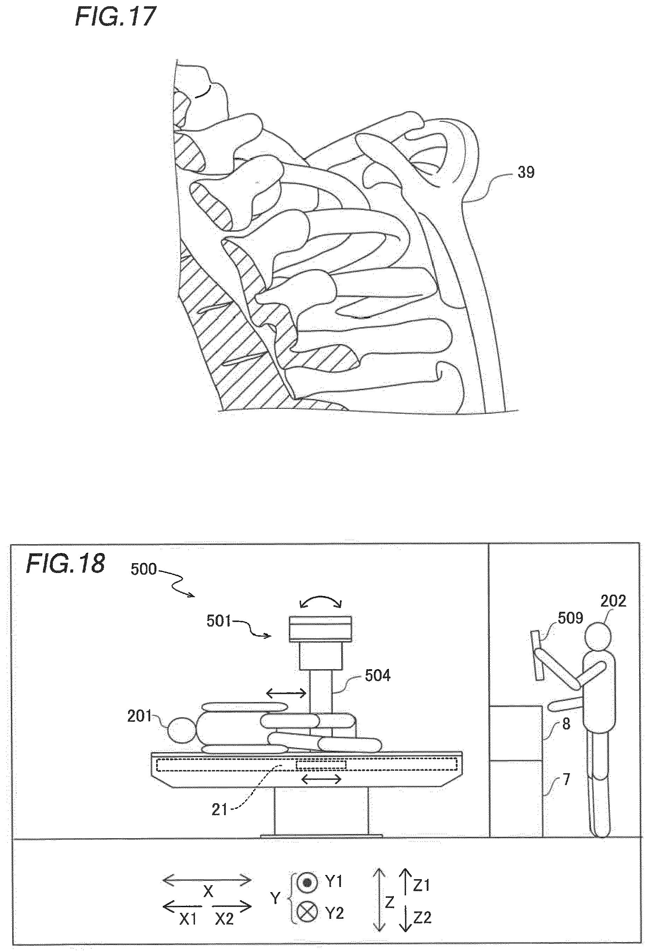

In the present invention, as shown in FIG. 17, the position correction information for correcting the imaging position may be acquired at the position where the outer edges of the plurality of predetermined portions of the scapula 39 are overlapped and an X-ray image in which the scapula 39 is imaged in the Y-shape can be captured when imaging a so-called scapula Y in which the scapula 39 is imaged in the Y-shape. The relative position of the X-ray irradiation unit with respect to the subject may be corrected based on the acquired position correction information. With this, it is possible to capture an X-ray image capable of accuracy identifying diseases around the scapula 39, such as, e.g., anterior dislocation, bone spines under the acromion, and calcification.

Further, in the above-described first to fourth embodiments, an example is shown in which the X-ray imaging apparatus 100 (600, 800, and 900) performs the calculation of the position correction information and the correction of the imaging position based on the position correction information, when imaging the knee joint (the knee side of the femur 31) and imaging the elbow joint (the knee side of the humerus 36), but the present invention is not limited thereto.

In the present invention, it may be configured such that the X-ray imaging apparatus calculates the position correction information when imaging other joints, such as, e.g., a hip joint, a finger joint, a wrist joint, and an ankle joint, and other site, and corrects the imaging position based on the position correction information.

Further, in the the above-described first to fourth embodiments, an example is shown in which the X-ray imaging apparatus 100 (600, 800, and 900) acquires the position correction information when imaging the knee joint (the knee side of the femur 31) and the elbow joint (the elbow side of the humerus 36), displays (notifies) the acquired position correction information, and control the change of the relative position of the X-ray irradiation unit 1 based on the acquired the acquired position correction information, but the present invention is not limited thereto.

In the present invention, the X-ray imaging apparatus may be configured to acquire the position correction information only at the time of imaging the knee joint (the knee side of the femur) and at the time of imaging the elbow joint (the elbow side of the humerus), performs the notification of the acquired position correction information, or perform the control to change the relative position of the X-ray irradiation unit based on the acquired position correction information. That is, the X-ray imaging apparatus may acquire the position correction information only at a particular imaging site, and performs the notification of the acquired position correction information or the control to change the relative position of the X-ray irradiation unit based on the acquired position correction information.

Further, in the above-described first to fourth embodiments, an example is shown in which the processing unit 81, 681, 881, and 981 (correction information acquisition unit) is configured to acquire the position correction information based on the positional relation between the outer edges of the plurality of predetermined portions of the bone of the subject 201 in the pre-shot image 24 (pre-position-correction image) generated based on the X-ray irradiation with less dose than when imaging the main shot image 23 (post-position correction image) captured after correcting the relative position of the X-ray irradiation unit 1 with respect to the bone of the subject 201, but the present invention is not limited thereto.

In the present invention, the image used for acquiring the position correction information by the correction information acquisition unit may be an X-ray image generated based on the irradiation of X-rays with a radiation dose greater than or equal to the radiation dose at the time of capturing the post-position-correction image to be captured after correcting the relative position of the X-ray irradiation unit. Further, the image to be used to acquire the position correction information by the correction information acquisition unit may be an image after performing preprocessing as described in the first to fourth embodiments on the X-ray image generated based on the the X-ray irradiation with a radiation dose equal to or more than that at the time of capturing a post-position-correction image captured after correcting the relative position of the X-ray irradiation unit.

Further, in the above-described first to fourth embodiments, an example is shown in which the processing unit 81, 681, 881, and 981 (correction information acquisition unit) is configured to input an X-ray image to the trained models 82a and 82b to acquire the outer edges of the plurality of predetermined portions based on the input X-ray image, and calculate the position correction information based on the acquired outer edges of the plurality of predetermined portions, but the present invention is not limited thereto.

In the present invention, the outer edges of the plurality of predetermined portions to be used to calculate the deviation amount of the medial condyle and the lateral condyle of the femur may be acquired (identified) by an image processing algorithm, such as an algorithm for extracting the feature points of the outer edges of the plurality of predetermined portions. Further, even in the case of acquiring the parameter information for acquiring parameters inherent to a subject, image processing algorithm that extracts feature points may be used instead of trained models.

Further, in the above-described first to fourth embodiments, an example is shown in which the processing unit 81, 681, 881, and 981 (correction information acquisition unit) acquires the outer edges of the plurality of predetermined portions based on the input X-ray image, and calculates the position correction information based on the acquired outer edges of the plurality of predetermined portions, but the present invention is not limited thereto.

In the present invention, the correction information acquisition unit may use, in addition to the X-ray image, a visible-light image of the subject captured by an optical camera that detects visible light to acquire the outer edges of the plurality of predetermined portions of the bone or the artificial joint (imaging target) of the subject and calculate the position correction information.

Further, in the above-described first to fourth embodiments, an example is shown in which the X-ray imaging apparatus 100 (600, 800, and 900) is configured to switch the mode for correcting the imaging position between an automatic correction mode and a manual correction mode based on the switching operation by the user 202, but the present invention is not limited thereto. In the present invention, the correction of the imaging position using the X-ray imaging apparatus may be performed only by either the automatic correction or the manual correction.

Further, in the above-described first to fourth embodiments, an example is shown in which the X-ray imaging apparatus 100 (600, 800, and 900) is configured to capture an image of the X-ray image by the X-rays emitted from the X-ray irradiation unit 1 suspended from the ceiling, but the present invention is not limited thereto.

As shown in FIG. 18, the present invention may be applied to an X-ray imaging apparatus 500 provided with an irradiation unit support mechanism 504 extending along the direction (Z-direction) intersecting the floor and an X-ray irradiation unit 501 supported by the irradiation unit support mechanism 504. That is, the X-ray imaging apparatus may be configured to capture an image of an X-ray image by the X-rays emitted from the X-ray irradiation unit 501 attached to the irradiation unit support mechanism 504 extending along the Z-direction intersecting the floor.

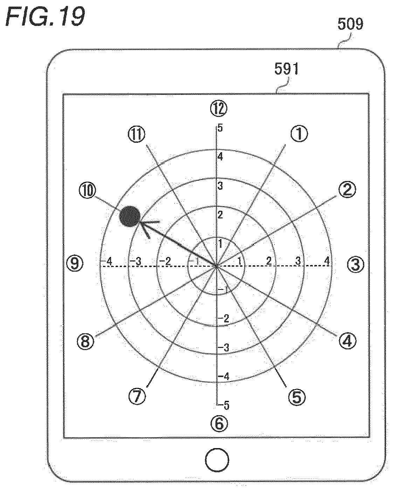

Further, in the correction of the imaging position using the X-ray imaging apparatus 500, as shown in FIG. 19, the position correction information is displayed on the display unit 591 of the operation terminal 509, and the correction of the imaging position is performed by the manual operation by the user 202. Note that the display unit 591 of the operation terminal 509 is provided with a touch panel for accepting the input operation of the user 202.

Further, in the above-described second to fourth embodiments, the slope $\alpha$, which is the coefficient for calculating the position correction information, is calculated based on the automatically acquired parameter information, but the present invention is not limited thereto.

In the present invention, the parameter information may be acquired based on the input operation by the user. Further, the parameter information may be calculated from the X-ray image, the appearance image, or the like by a processing apparatus different from the processing unit 681, 881, and 981 (correction information acquisition unit) for calculating the position correction information. Further, the parameter information set (stored) in advance may be acquired from an external device, such as, e.g., a server. Further, the parameter information may include a value of the subject inherent parameter (e.g., the height b and the distance D) for calculating the position correction information.

Further, in the above-described second embodiment, an example is shown in which as the parameter information, the size of the portion 31c and the portion 31d of the femur 31 o and the size of the portion 32c of the tibia 32 f the subject 201 are detected as the size of the detection target portion of the subject 201, but the present invention is not limited thereto. In the present invention, the size of at least one of three of the portion 31c, the portion 31d of the femur 31, and the portion 32c of the tibia 32 may be acquired as the parameter information. Further, when performing the X-ray imaging of the knee, the size of the portion other than the portion 31c, the portion 31d, and the portion 32c may be detected as the size of the detection target portion of the subject.

In the above-described third embodiment, an example is shown in which the physique information indicating the length of the leg of the subject 201 is acquired as the parameter information, but the present invention is not limited thereto. In the present invention, the height of the entire body of the subject may be detected, and the physique information indicating the detected height of the subject may be acquired as the parameter information. In the case of X-ray imaging the elbow joint, the physique information indicating the length of the arm may be acquired as the parameter information. Further, the physique information indicating the shoulder width, the body thickness, and the like may be acquired as the parameter information.

Further, in the above-described third embodiment, an example is shown in which the processing of acquiring the parameter information from the appearance image 25 by the image processing unit 812 which is a module different from the processing unit 81 (correction information acquisition unit) for calculating the position correction information is performed. However, the parameter information may be acquired by outputting the appearance image by the imaging unit to the correction information acquisition unit so that the correction information acquisition unit detects the physique information from the appearance image to acquire the parameter information.

Further, in the above-described third embodiment, an example is shown in which the subject 201 lying on the top board 3 is imaged by the imaging unit 811 arranged on the X-ray irradiation unit 1, but the present invention is not limited thereto. In the present invention, the imaging unit may be located at the ceiling portion of the examination room instead of the X-ray irradiation unit. Further, the parameter information may be acquired by capturing an image of subject in a standing posture instead of the subject lying on the top board.

Further, in the above-described first to fourth embodiments, for convenience of explanation, the processing of correcting (automatically correcting and manually correcting) the imaging position using the X-ray imaging apparatus according to the present invention is described using a flow-driven flowchart that sequentially performs the processing according to the processing flow, but the present invention is not limited thereto. In the present invention, the processing of correcting the imaging position using the X-ray imaging apparatus may be performed by event-driven processing (event-driven type) that executes the processing on an event-by-event basis. In this case, the processing may be performed in a complete event-driven fashion or in combination of event-driven type processing and flow-driven type processing.

**[Aspects]**

It will be understood by those skilled in the art that the above-described exemplary embodiments are concrete examples of the following aspects.

**(Item** 1)

An X-ray imaging apparatus comprising:

an X-ray irradiation unit configured to irradiate a subject with X-rays;
an X-ray detection unit configured to detect the X-rays emitted from the X-ray irradiation unit and transmitted through the subject; and
a correction information acquisition unit configured to identify an outer edge of each of a plurality of predetermined portions of one imaging target of the subject in an X-ray image captured based on a direction signal of the X-ray detection unit, and acquire position correction information for correcting a relative position of the X-ray irradiation unit with respect to the imaging target of the subject, based on a positional relation between the identified outer edges of the plurality of the predetermined portions,

wherein the position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged with a predetermined positional relation can be captured.

**(Item 2)**

The X-ray imaging apparatus as recited in the above-described Item 1,
wherein at least one of a notification of the position correction information acquired by the correction information acquisition unit and control to change the relative position of the X-ray irradiation unit with respect to the imaging target of the subject based on the position correction information is performed.

**(Item 3)**

The X-ray imaging apparatus as recited in the above-described Item 2, further comprising:

a movement mechanism configured to change the relative position of the X-ray irradiation unit with respect to the subject; and
a movement control unit configured to control to change the relative position of the X-ray irradiation unit with respect to the subject by the movement mechanism,
wherein the movement control unit is configured to control to change the relative position of the X-ray irradiation unit with respect to the imaging target of the subject by the movement mechanism, based on the position correction information acquired by the correction information acquisition unit.

**(Item 4)**

The X-ray imaging apparatus as recited in the above-described Item 3, further comprising:

a top board configured to place the subject thereon,
wherein the movement control unit controls to automatically move a position of at least one of the X-ray irradiation unit and the top board based on the position correction information acquired by the correction information acquisition unit such that the relative position of the X-ray irradiation unit with respect to the imaging target of the subject becomes a position where the X-ray image in which the positional relation between the outer edges of the plurality of predetermined portions is image with the predetermined positional relation can be captured.

**(Item 5)**

The X-ray imaging apparatus as recited in the above-described Item 3, further comprising:

a top board configured to place the subject thereon,
wherein the movement control unit controls to restrict a movement of one of the X-ray irradiation unit and the top board, based on the position correction information acquired by the correction information acquisition unit when moving one of the X-ray irradiation unit and the top board based on an operation of the user.

**(Item 6)**

The X-ray imaging apparatus as recited in claim 2, further comprising:

a display unit,
wherein the display unit displays, as a notification of the position correction information acquired by the correction information acquisition unit, at least a relative moving direction of the X-ray irradiation unit out of the relative moving direction and the movement amount of the X-ray irradiation unit with respect to the imaging target of the subject for correcting such that the X-ray image in which the positional relation between the outer edges of the plurality of predetermined portions is imaged with the predetermined positional relation can be captured.

**(Item 7)**

The X-ray imaging apparatus as recited in any one of the above-described Items 2 to 6,

wherein the correction information acquisition unit is configured to
acquire a relative positional deviation between the outer edges of the plurality of predetermined portions in the X-ray image based on the positional relation between the outer edges of the plurality of predetermined portions in one imaging target of the subject in the X-ray image, and
acquire the position correction information based on the acquired relative positional deviation between the outer edges of the plurality of predetermined portions.

**(Item 8)**

The X-ray imaging apparatus as recited in any one of the above-described Items 2 to 7,

wherein the correction information acquisition unit is configured to
identify the outer edge of each of the plurality of predetermined portions of a bone or an artificial joint as one imaging target of the subject in the X-ray image, and
acquire the position correction information for correcting the relative position of the X-ray irradiation unit with respect to the bone or the artificial joint of the subject, based on the positional relation between the identified outer edges of the plurality of predetermined portions of the bone or the artificial joint.

**(Item 9)**

The X-ray imaging apparatus as recited in the above-described Item 8,

wherein the correction information acquisition unit is configured to
acquire the relative positional deviation between the outer edges of the plurality of predetermined portions, based on a degree of overlap between the outer edges of the plurality of predetermined portions in each of the bone or the artificial joint of the subject in the X-ray image, and
calculate the position correction information for correcting the relative position of the X-ray irradiation unit with respect to the bone or the artificial join of the subject to a position where the X-ray image in which the outer edges of the plurality of predetermined portions are imaged in an overlapped manner can be captured.

**(Item 10)**

The X-ray imaging apparatus as recited in the above-described Item 9,
wherein the correction information acquisition unit is configured to calculate the position correction information for correcting the relative position of the X-ray irradiation with respect to the bone or the artificial joint of the subject to a position where the X-ray image in which the outer edges of the plurality of predetermined portions are imaged in an overlapped manner can be captured, when imaging at least one of a knee side of a femur and a scapula of the subject.

**(Item 11)**

The X-ray imaging apparatus as recited in any one of the above-described Items 8 to 10,

wherein the correction information acquisition unit is configured to
acquire the relative positional deviation between the outer edges of the plurality of the predetermined portions, based on the positional relation between the outer edges of the plurality of predetermined portions in each of the bone or the artificial joint of the subject in the X-ray image, and
calculate the position correction information for correcting the relative position of the X-ray irradiation with respect to the bone or the artificial joint of the subject to a position where the X-ray image in which the outer edges of the plurality of predetermined positions are imaged concentrically can be captured.

**(Item 12)**

The X-ray imaging apparatus as recited in the above-described Item 11,
wherein the correction information acquisition unit is configured to calculate the position correction information for correcting the relative position of the X-ray irradiation unit with respect to the bone or the artificial joint of the subject to a position where an image in which the outer edges of the plurality of predetermined portions are imaged concentrically can be captured, when imaging an elbow side of a humerus of the subject.

**(Item 13)**

The X-ray imaging apparatus as recited in any one of the above-described Items 1 to 12,
wherein the correction information acquisition unit is configured to acquire the position correction information, based on the positional relation between the outer edges of the plurality of predetermined portions in the imaging target of the subject in a pre-position-correction image which is the X-ray image generated based on X-ray irradiation with less radiation dose than that at the time of capturing a post-position-correction image which is the X-ray image captured after correcting the relative position of the X-ray irradiation unit with respect to the imaging target of the subject.

**Item 14**

The X-ray imaging apparatus as recited in any one of the above-described Items 1 to 13,

wherein the correction information acquisition unit is configured to
input the X-ray image to trained models in which the X-ray image capturing the imaging target is machine trained as input data to acquire the outer edges of the plurality of predetermined portions based on the input X-ray image, and calculate the position correction information based on the acquired outer edges of the plurality of predetermined portions.

**(Item 15)**

The X-ray imaging apparatus as recited in the above-described Items 1 to 14,
wherein the correction information acquisition unit is configured to calculate the position correction information, based on parameter information corresponding to an actual distance between the plurality of predetermined portions in the imaging target of the subject.

**(Item 16)**

The X-ray imaging apparatus as recited in the above-described Item 15,
wherein the correction information acquisition unit detects, as the parameter information, a size of the detection target portion of the subject in the X-ray image of the subj ect.

**(Item 17)**

The X-ray imaging apparatus as recited in the above-described Item 16,
wherein the correction information acquisition unit detects, as the parameter information, the size of the detection target portion of the subject from the X-ray image captured to acquire the position correction information.

**(Item 18)**

The X-ray imaging apparatus as recited in the above-described Item 16,
wherein the correction information acquisition unit detects, as the parameter information, the size of the the detection target portion of the subject from the X-ray image captured in advance at an imaging angle different from the X-ray image for acquiring the position correction information.

**(Item 19)**

The X-ray imaging apparatus as recited in the above-described Item 15, further comprising:

an imaging unit configured to image an appearance of the subject,
wherein the correction information acquisition unit is configured to calculate the position correction information, based on the parameter information which is physique information indicating a size of a physique of the subject calculated from an appearance image of the subject captured by the imaging unit.

**(Item 20)**

An imaging position correction method comprising:

an irradiation step of irradiating a subject with X-rays from an X-ray irradiation unit;
a detection step of detecting X-rays transmitted through the subject; and
a correction information acquisition step,
wherein the correction information acquisition step identifies an outer edge of each of a plurality of predetermined portions in one imaging target of the subject in an X-ray image captured based on a direction of the X-rays in the detection step, and acquires position correction information for correcting a relative position of the X-ray irradiation unit with respect to the imaging target of the subject, based on a positional relation between identified outer edges of the plurality of predetermined portions, and
wherein the position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged with a predetermined positional relation can be captured.

**(Item 21)**

The imaging position correction method as recited in the above-described Item 20, further comprising:

a position correction step,
wherein the position correction step performs at least one of a notification of the position correction information acquired in the correction information acquisition step and control to change the relative position of the X-ray irradiation unit with respect to the imaging target of subject, based on the position correction information.

**(Item 22)**

The imaging position correction method as recited in the above-described Item 20 or 21,
wherein the correction information acquisition step includes the correction information acquisition step for calculating the position correction information, based on parameter information corresponding to an actual distance between the plurality of predetermined portions in the imaging target of thee subject.

**Claims**

1.  An X-ray imaging apparatus (100, 500, 600, 800, 900) comprising:

an X-ray irradiation unit (1, 501) configured to irradiate a subject (201) with X-rays;
an X-ray detection unit (2) configured to detect the X-rays emitted from the X-ray irradiation unit and transmitted through the subject; and
a correction information acquisition unit (81, 681, 881, 981)configured to identify an outer edge of each of a plurality of predetermined portions of one imaging target of the subject in an X-ray image captured based on a direction signal of the X-ray detection unit, and acquire position correction information for correcting a relative position of the X-ray irradiation unit with respect to the imaging target of the subject, based on a positional relation between the identified outer edges of the plurality of the predetermined portions,
wherein the position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged with a predetermined positional relation can be captured.

2.  The X-ray imaging apparatus as recited in claim 1,
wherein at least one of a notification of the position correction information acquired by the correction information acquisition unit and control to change the relative position of the X-ray irradiation unit with respect to the imaging target of the subject based on the position correction information is performed.

3.  The X-ray imaging apparatus as recited in claim 2, further comprising:

a movement mechanism (4, 5) configured to change the relative position of the X-ray irradiation unit with respect to the subject; and
a movement control unit (7) configured to control to change the relative position of the X-ray irradiation unit with respect to the subject by the movement mechanism,

wherein the movement control unit is configured to control to change the relative position of the X-ray irradiation unit with respect to the imaging target of the subject by the movement mechanism, based on the position correction information acquired by the correction information acquisition unit.

4. The X-ray imaging apparatus as recited in claim 3, further comprising:

a top board (3) configured to place the subject thereon,
wherein the movement control unit controls to automatically move a position of at least one of the X-ray irradiation unit and the top board based on the position correction information acquired by the correction information acquisition unit such that the relative position of the X-ray irradiation unit with respect to the imaging target of the subject becomes a position where the X-ray image in which the positional relation between the outer edges of the plurality of predetermined portions is image with the predetermined positional relation can be captured.

5. The X-ray imaging apparatus as recited in claim 3, further comprising:

a top board configured to place the subject thereon,
wherein the movement control unit controls to restrict a movement of one of the X-ray irradiation unit and the top board, based on the position correction information acquired by the correction information acquisition unit when moving one of the X-ray irradiation unit and the top board based on an operation of the user.

6. The X-ray imaging apparatus as recited in any one of claims 2 to 5, further comprising:

a display unit (91, 591),
wherein the display unit displays, as a notification of the position correction information acquired by the correction information acquisition unit, at least a relative moving direction of the X-ray irradiation unit out of the relative moving direction and the movement amount of the X-ray irradiation unit with respect to the imaging target of the subject for correcting such that the X-ray image in which the positional relation between the outer edges of the plurality of predetermined portions is imaged with the predetermined positional relation can be captured.

7. The X-ray imaging apparatus as recited in any one of claims 2 to 6,

wherein the correction information acquisition unit is configured to
acquire a relative positional deviation between the outer edges of the plurality of predetermined portions in the X-ray image based on the positional relation between the outer edges of the plurality of predetermined portions in one imaging target of the subject in the X-ray image, and
acquire the position correction information based on the acquired relative positional deviation between the outer edges of the plurality of predetermined portions.

8. The X-ray imaging apparatus as recited in any one of claims 2 to 7,

wherein the correction information acquisition unit is configured to
identify the outer edge of each of the plurality of predetermined portions of a bone or an artificial joint as one imaging target of the subject in the X-ray image, and
acquire the position correction information for correcting the relative position of the X-ray irradiation unit with respect to the bone or the artificial joint of the subject, based on the positional relation between the identified outer edges of the plurality of predetermined portions of the bone or the artificial joint.

9. The X-ray imaging apparatus as recited in claim 8,

wherein the correction information acquisition unit is configured to
acquire the relative positional deviation between the outer edges of the plurality of predetermined portions, based on a degree of overlap between the outer edges of the plurality of predetermined portions in each of the bone or the artificial joint of the subject in the X-ray image, and
calculate the position correction information for correcting the relative position of the X-ray irradiation unit with respect to the bone or the artificial join of the subject to a position where the X-ray image in which the outer edges of the plurality of predetermined portions are imaged in an overlapped manner can be captured.

10. The X-ray imaging apparatus as recited in claim 9,

wherein the correction information acquisition unit is configured to calculate the position correction information for correcting the relative position of the X-ray irradiation with respect to the bone or the artificial joint of the subject to a position where the X-ray image in which the outer edges of the plurality of predetermined portions are imaged in an overlapped manner can be captured, when imaging at least one of a knee side of a femur (31) and a scapula (39) of the subject.

11. The X-ray imaging apparatus as recited in any one of claims 8 to 10,

wherein the correction information acquisition unit is configured to
acquire the relative positional deviation between the outer edges of the plurality of the predetermined portions, based on the positional relation between the outer edges of the plurality of predetermined portions in each of the bone or the artificial joint of the subject in the X-ray image, and
calculate the position correction information for correcting the relative position of the X-ray irradiation with respect to the bone or the artificial joint of the subject to a position where the X-ray image in which the outer edges of the plurality of predetermined positions are imaged concentrically can be captured.

12. The X-ray imaging apparatus as recited in claim 11,
wherein the correction information acquisition unit is configured to calculate the position correction information for correcting the relative position of the X-ray irradiation unit with respect to the bone or the artificial joint of the subject to a position where an image in which the outer edges of the plurality of predetermined portions are imaged concentrically can be captured, when imaging an elbow side of a humerus (36) of the subject.

13. The X-ray imaging apparatus as recited in any one of claims 1 to 12,
wherein the correction information acquisition unit is configured to acquire the position correction information, based on the positional relation between the outer edges of the plurality of predetermined portions in the imaging target of the subject in a pre-position-correction image (24, 24a, 24b) which is the X-ray image generated based on X-ray irradiation with less radiation dose than that at the time of capturing a post-position-correction image (23) which is the X-ray image captured after correcting the relative position of the X-ray irradiation unit with respect to the imaging target of the subject.

14. The X-ray imaging apparatus as recited in any one of claims 1 to 13,

wherein the correction information acquisition unit is configured to
input the X-ray image to trained models (82a, 82b, 82c, 82d, 82e) in which the X-ray image capturing the imaging target is machine trained as input data to acquire the outer edges of the plurality of predetermined portions based on the input X-ray image, and
calculate the position correction information based on the acquired outer edges of the plurality of predetermined portions.

15. The X-ray imaging apparatus as recited in any one of claims 1 to 14,
wherein the correction information acquisition unit is configured to calculate the position correction information, based on parameter information corresponding to an actual distance between the plurality of predetermined portions in the imaging target of the subject.

16. The X-ray imaging apparatus as recited in claim 15,
wherein the correction information acquisition unit detects, as the parameter information, a size of the detection target portion (31c, 31d, 32c) of the subject in the X-ray image of the subject.

17. The X-ray imaging apparatus as recited in claim 16,
wherein the correction information acquisition unit detects, as the parameter information, the size of the detection target portion of the subject from the X-ray image (24) captured to acquire the position correction information.

18. The X-ray imaging apparatus as recited in claim 16,
wherein the correction information acquisition unit detects, as the parameter information, the size of the the detection target portion of the subject from the X-ray image (26) captured in advance at an imaging angle different from the X-ray image (24) for acquiring the position correction information.

19. The X-ray imaging apparatus as recited in claim 15, further comprising:

an imaging unit (811) configured to image an appearance of the subject,
wherein the correction information acquisition unit is configured to calculate the position correction information, based on the parameter information which is physique information indicating a size of a physique of the subject calculated from an appearance image (25) of the subject captured by the imaging unit.

20. An imaging position correction method comprising:

an irradiation step of irradiating a subject with X-rays from an X-ray irradiation unit;
a detection step of detecting X-rays transmitted through the subject; and
a correction information acquisition step,
wherein the correction information acquisition step identifies an outer edge of each of a plurality of predetermined portions in one imaging target of the subject in an X-ray image captured based on a direction of the X-rays in the detection step, and acquires position correction information for correcting a relative position of the X-ray irradiation unit with respect to the imaging target of the subject, based on a positional relation between identified outer edges of the plurality of predetermined portions, and
wherein the position correction information includes a relative moving direction and a movement amount for correcting the relative position of the X-ray irradiation unit with respect to the imaging target of the subject to a position where the X-ray image in which the positional relation between the outer edges of the plurality of predetermined portions is imaged with a predetermined positional relation can be captured.

21. The imaging position correction method as recited in claim 20, further comprising:

a position correction step,
wherein the position correction step performs at least one of a notification of the position correction information acquired in the correction information acquisition step and control to change the relative position of the X-ray irradiation unit with respect to the imaging target of subject, based on the position correction information.

22. The imaging position correction method as recited in claim 20 or 21,
wherein the correction information acquisition step includes the correction information acquisition step for calculating the position correction information, based on parameter information corresponding to an actual distance between the plurality of predetermined portions in the imaging target of thee subject.

## FIG.1

## FIG.2

## FIG.3

## FIG.4

Right knee rear face

Origin of medial head of gastrocnemius muscle

Adductor tubercle

Epicondylus medialis

Medial plateau

Fossa intercondylaris

Epicondylus lateralis

Popliteal muscle tendon muscle

Outer plateau

Eminentia intercondylaris

FIG.5

FIG.6

## FIG.7

Calculation of deviation amount

24

82a Trained model

82b Trained model

Medial condyle image

Lateral condyle image

Estimation result

— Lateral condyle image
---- Medial condyle image

FIG.8

$f(x)$

Deviation
amount [f]

$(x_1, f_1)$

$f_1$

$0$

$x_1$

$(x', 0)$

$x' = -\dfrac{f_1}{\alpha}$

X-ray tube position [x]

FIG.9

34

31

35

32a

32b

32

33

FIG.10

FIG.11

⊗ X-ray irradiation point

FIG.12

⊗ X-ray irradiation point

FIG.13

Outer side

Inner side (body side)

Shoulder side

36

A

B

36a

36b

37

C

X-ray irradiation point

Elbow side

38

with deviation

36

37

38

Portion A
Portion B
Portion C

Appropriate

36

37

38

Portion A
Portion B
Portion C

FIG.14

Correction

Correction

Joint gap

## FIG.15

Automatic correction mode

Start

301 X-ray irradiation

302 X-ray detection

303 Acquisition of position correction information

304 Automatic position correction

End

## FIG.16

Manual correction mode

```
        ( Start )
           │
           ▼
┌──────────────────────┐  401
│   X-ray irradiation   │
└──────────────────────┘
           │
           ▼
┌──────────────────────┐  402
│    X-ray detection    │
└──────────────────────┘
           │
           ▼
┌──────────────────────┐  403
│  Acquisition of position │
│  correction information │
└──────────────────────┘
           │
           ▼
┌──────────────────────┐  404
│   Display of position  │
│  correction information │
└──────────────────────┘
           │
           ▼
┌──────────────────────┐  405
│   Manual position     │
│      correction       │
└──────────────────────┘
           │
           ▼
        (  End  )
```

FIG.17

FIG.18

EP 4 230 143 A1

FIG.19

42

## FIG.20

600

| | |
|---|---|
| X-ray irradiation unit (1) | Apparatus control unit (7) |
| X-ray detection unit (2) | Processing apparatus (608) |
| Irradiation unit movement mechanism (4) | Processing unit (681) |
| Top board movement mechanism (5) | Storage unit (82) |
| Detector movement mechanism (6) | Operation terminal (9) |

## FIG.21

*FIG.22*

```
                    ┌─────────────┐
                    │    Start    │
                    └──────┬──────┘
                           │
                           ▼
           ┌───────────────────────────────┐  701
           │       X-ray irradiation        │
           └───────────────┬───────────────┘
                           │
                           ▼
           ┌───────────────────────────────┐  702
           │        X-ray detection         │
           └───────────────┬───────────────┘
                           │
                           ▼
           ┌───────────────────────────────┐  703
           │        Acquisition of          │
           │      parameter information     │
           └───────────────┬───────────────┘
                           │
                           ▼
           ┌───────────────────────────────┐  704
           │        Acquisition of          │
           │  position correction information │
           └───────────────┬───────────────┘
                           │
                           ▼
           ┌───────────────────────────────┐  705
           │          Automatic             │
           │      position correction       │
           └───────────────┬───────────────┘
                           │
                           ▼
                    ┌─────────────┐
                    │     End     │
                    └─────────────┘
```

## FIG.23

800

808

| X-ray irradiation unit | 1 |
| X-ray detector | 2 |
| Irradiation unit movement mechanism | 4 |
| Top board movement mechanism | 5 |
| Detector movement mechanism | 6 |
| Apparatus control unit | 7 |

Processing apparatus

Processing unit — 881

Storage unit — 82

Operation terminal — 9

Imaging unit — 811

Image processing unit — 812

## FIG.24

25

201

201a

201b

## FIG.25

900

| | |
|---|---|
| X-ray irradiation unit    1 | Apparatus control unit    7 |
| X-ray detection unit    2 | Processing apparatus    908 |
| Irradiation unit movement mechanism    4 | Processing unit    981 |
| Top board movement mechanism    5 | Storage unit    82 |
| Detector movement mechanism    6 | Operation terminal    9 |

## FIG.26

Europäisches
Patentamt

European
Patent Office

Office européen
des brevets

## EUROPEAN SEARCH REPORT

Application Number

EP 23 15 7493

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/256134 A1 (NAKAMURA KEIGO [JP]) 13 September 2018 (2018-09-13) | 1,2,6-9, 13,20,21 | INV. A61B6/00 |
| Y | * paragraph [0039] – paragraph [0066]; figures * | 3-5,8, 10-12, 14,15,22 | G06T7/11 |
| A | | 16-19 | |
| | ----- | | |
| Y | US 2021/192731 A1 (YAMAMURA TAKUYA [JP]) 24 June 2021 (2021-06-24) | 8,10,14, 15,22 | |
| A | * paragraph [0063] – paragraph [0199]; figures * | 16-19 | |
| | ----- | | |
| Y | US 2019/142356 A1 (NAKAYA TOMOHIRO [JP]) 16 May 2019 (2019-05-16) | 3-5,11, 12 | |
| A | * paragraph [0108] – paragraph [0139]; figures * | 16-19 | |
| | ----- | | |

**TECHNICAL FIELDS SEARCHED (IPC)**

A61B
G06T

The present search report has been drawn up for all claims

| Place of search | Date of completion of the search | Examiner |
|---|---|---|
| Munich | 21 June 2023 | Strubel, Christine |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons
...................................................................................................
& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 23 15 7493

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2023

| Patent document cited in search report | Publication date | Patent family member(s) | Publication date |
|---|---|---|---|
| US 2018256134    A1 | 13-09-2018 | JP       6845047 B2<br>JP   2018143699 A<br>US   2018256134 A1 | 17-03-2021<br>20-09-2018<br>13-09-2018 |
| US 2021192731    A1 | 24-06-2021 | JP   2021097864 A<br>US   2021192731 A1 | 01-07-2021<br>24-06-2021 |
| US 2019142356    A1 | 16-05-2019 | CN       106793983 A<br>JP       6264589 B2<br>JP WO2016051603 A1<br>US   2019142356 A1<br>WO   2016051603 A1 | 31-05-2017<br>24-01-2018<br>27-04-2017<br>16-05-2019<br>07-04-2016 |

EPO FORM P0459

For more details about this annex : see Official Journal of the European Patent Office, No. 12/82

**EP 4 230 143 A1**

## REFERENCES CITED IN THE DESCRIPTION

**Patent documents cited in the description**

- JP 2014117368 A